# EUROPEAN PATENT SPECIFICATION

(11) **EP 1 686 171 B1**
(45) Date of publication and mention of the grant of the patent: **05.08.2015**
(21) Application number: 04792798.3
(22) Date of filing: 15.10.2004
(51) Int. Cl.: C12N 5/00, C12M 3/00, G03F 7/20, A61L 27/00

(54) **METHOD OF CONSTRUCTING ARTIFICIAL CELL TISSUE AND BASE MATERIAL THEREFOR**
VERFAHREN ZUR KONSTRUKTION VON KÜNSTLICHEM ZELLGEWEBE UND GRUNDMATERIAL DAFÜR
PROCEDE D'ELABORATION DE TISSU CELLULAIRE ARTIFICIEL, ET MATERIAU DE BASE CORRESPONDANT

(30) Priority: 17.10.2003 JP 2003358397
(43) Date of publication of application: 02.08.2006
(73) Proprietor: DAI NIPPON PRINTING CO., LTD., Tokyo 162-8001 (JP)
(72) Inventor: MORITA, Ikuo, Tokyo 174-0046 (JP); NAKAMURA, Makoto, 162-8001, Tokyo (JP); MIYAKE, Hideyuki, Tokyo 162-8001 (JP); HATTORI, Hideshi, Tokyo 162-8001 (JP); KOBAYASHI, Hironori, Tokyo 162-8001 (JP); KURIHARA, Masaaki, Tokyo 162-8001 (JP)
(74) Representative: Denison, Christopher Marcus
(86) International application number: PCT/JP2004/015656
(87) International publication number: WO 2005/038011

(56) References cited:
- EP-A- 1 246 011
- EP-A- 1 260 863
- EP-A1- 1 199 354
- JP-A- 2002 542 883
- JP-A- 2003 039 399
- JP-A- 2003 228 172
- JP-A- 2004 344 025
- US-A- 5 721 131
- KOBAYASHI AKIKO ET AL: "In vitro formation of capillary networks using optical lithographic techniques." BIOCHEMICAL AND BIOPHYSICAL RESEARCH COMMUNICATIONS 6 JUL 2007, vol. 358, no. 3, 6 July 2007 (2007-07-06), pages 692-697, XP002441699 ISSN: 0006-291X

## Description

### Technical Field

The present invention relates to a method for culturing cells in a patterned state, and a substrate to which cells have adhered in a patterned state for use in a method of treatment.

### Background Art

Recently, technology for direct transplantation of artificial alternates or cell tissues obtained by culturing cells has been a focus of attention. Typical examples of such technology include artificial skin, artificial blood vessels, and cultured cell tissues. Artificial skin or the like containing a synthetic polymer is not preferable for transplantation because it may cause rejection or other problems. On the other hand, with a cultured cell tissue, there is no concern about rejection, because such tissue is obtained by culturing the cells of a subject into which the tissue will be transplanted and thus it is preferable for transplantation. Such cultured cell tissue is prepared by collecting cells from a subject for transplantation and then culturing the cells.

Many animal cells have adhesion dependency (cells grow while adhering to something). Animal cells are thus unable to survive for a long time period in a floating state *ex vivo.* In a cell culture for preparing the above cell tissue, for example, a polymer material such as modified polystyrene having enhanced cell adhesiveness through surface treatment or a culture plate which is prepared by uniformly applying a cell adhesive protein (e.g., collagen and fibronectin) to glass or a polymer material has been used as a carrier. Cells that have adhered to such carrier in a planar state can be cultured successfully. However, such cells generally form a tissue with difficulty, so that it is impossible to obtain original cellular functions. For example, an example of a study report is that the albumin-producing ability of cultured hepatocytes that have not yet formed any tissue decreases to a fraction of that of hepatocytes that have formed a tissue (liver spheroid).

In contrast, another technology that has been reported comprises, causing cultured cells to adhere to only a fine part on a substrate, so as to array the cells thereby promoting cell tissue formation. A method used for arraying cultured cells comprises culturing cells on the surface of a substrate in which surfaces each having different cellular adhesion properties form a pattern and causing cells to adhere to only the surface that has been processed to adhere the cells, thereby arraying the cells.

For example, JP Patent Publication (Kokai) No. 2-245181 A (1990) discloses the application of a charge-retaining medium having an electrostatic charge pattern formed thereon to cell culture for the purpose of growing nerve cells in a circuit form. Furthermore, JP Patent Publication (Kokai) No. 3-7576 A (1991) discloses an attempt to array cultured cells on a surface on which a non-cell-adhesive or cell adhesive photosensitive hydrophilic polymer has been patterned using a photolithography method.

Furthermore, JP Patent Publication (Kokai) No. 5-176753 A (1993) discloses a cell culture substrate on which a substance such as collagen that affects cell adhesion rate or cell morphology has been patterned, and a method for preparing such substrate using a photolithography method. Culture of cells on such substrate enables adhesion of a greater number of cells to a surface on which collagen or the like has been patterned and cell patterning.

However, it may be necessary for such pattern for cell culture to be very fine depending on the application. A high-definition pattern can be obtained when the above-described patterning is carried out bya photolithography method or the like using a photosensitive material. In such case, however, a cell adhesive material should have photosensitivity. Chemical modification of a biopolymer or the like to impart such photosensitivity is often difficult. This leads to a problem such that the selectivity range of a cell adhesive material is extremely narrowed. A photolithography method using a photoresist requires the use of a developing solution or the like that may adversely affect cell culture. Moreover, biomaterials and the like having high ability to culture cells are generally difficult to decompose by plasma. Thus, patterning using a plasma etching method also has low industrial productivity and thus is impractical.

Furthermore, the above patterned and cultured cells are harvested through treatment with proteinase such as trypsin or a chemical drug. Thus, such process is problematic in that treatment steps become complicated, the possibility of contamination becomes high, cells are denatured or damaged, original cellular functions may deteriorate, and the like.

Accordingly, JP Patent Publication (Kokai) No. 2003-38170 A discloses a method for producing a cell sheet, which comprises preparing a cell culture support obtained by patterning a temperature-responsive polymer on a substrate, culturing cells on the cell culture support, causing the support to closely adhere to a polymer film by varying temperature, and removing the cells together with the polymer film from the support without damaging the cells, so as to produce a cell sheet. However, it is difficult to efficiently form a fine pattern using the method disclosed herein. Furthermore, the method also requires variation of temperature when cells are removed from the support, so that the steps are complicated. Moreover, direct patterning on a biomaterial such as an organ is also difficult.

Furthermore, a method for constructing an artificial organ using a specific cell culture method is also known (JP Patent Publication (Kokai) 2003-24351 A). With this method, an artificial blood vessel is formed by adhesion of vascular endothelial cells or the like to a tubular cell culture substrate. However, in order to prepare many artificial blood vessels by this method, many finely processed cell culture substrates must be prepared and tissue formation requires much time. Thus, such method has also low industrial productivity.

EP 1 199 354 A1 refers to cells cultured on a patterned substrate. The grown cells are then embedded in a matrix and said matrix containing the cells is then removed from the substrate.

### Disclosure of the Invention

The present invention has been completed to address the above problems in conventional technology. Specifically, an object of the present invention is to array and culture cells in a finely patterned state using a convenient method without damaging the cells and thus to promote tissue formation by cultured cells.

As a result of intensive studies to achieve the above object, the present inventors have discovered that cells can be cultured in an arrayed state by inoculating cells on a cell array substrate, on which regions having cell adhesiveness have been patterned, causing the cells to adhere to the substrate in a pattern so as to array the cells, and then transferring the thus arrayed cells to a cell culture substrate. Thus, the present inventors have completed the present invention.

The present invention relates to an in vitro or ex vivo method for culturing cells, which comprises the steps of: causing cells to adhere to the surface of a cell array substrate having a cell adhesiveness variation pattern that comprises regions having good cell adhesiveness and regions having inhibited cell adhesiveness patterned on a substrate; transferring the adhered cells to a cell culture substrate in such patterned state; and culturing the transferred cells, wherein

the regions having good cell adhesiveness in the cell adhesiveness variation pattern have water contact angles between 10° and 40°.

In an embodiment of the cell culture method of the present invention, the cell adhesiveness variation pattern is formed of a cell adhesiveness variation layer that comprises a cell adhesiveness variation material whose cell adhesiveness is varied by the action of a photocatalyst along with energy irradiation.

In an embodiment of the cell culture method of the present invention, the cell adhesiveness variation layer is a photocatalyst-comprising cell adhesiveness variation layer that comprises a photocatalyst and the cell adhesiveness variation material.

In an embodiment of the cell culture method of the present invention, the cell adhesiveness variation layer comprises a photocatalyst-comprising photocatalyst treatment layer and a cell adhesiveness variation material layer that comprises the cell adhesiveness variation material formed on the photocatalyst treatment layer.

In an embodiment of the cell culture method of the present invention, the cell adhesiveness variation pattern is formed by arranging the cell adhesiveness variation layer that comprises the cell adhesiveness variation material and the photocatalyst-comprising layer so that the layers face each other, and then carrying out energy irradiation.

In an embodiment of the cell culture method of the present invention, the cell culture substrate is made of a biomaterial. In an embodiment of the cell culture method of the present invention, the cell adhesiveness variation pattern is a pattern wherein linear regions having good cell adhesiveness are arranged on regions having inhibited cell adhesiveness. In an embodiment of the cell culture method of the present invention, the cell adhesiveness variation pattern is a pattern wherein linear regions having good cell adhesiveness and spaces of the regions having inhibited cell adhesiveness are arranged alternately. In such embodiment, the line widths of the regions having good cell adhesiveness are each between 20 µm and 200 µm, the space widths between such lines are each between 300 µm and 1000 µm, and the cells used are vascular endothelial cells. The present application also describes a cell tissue that is formed by the above cell culture method. The present invention further relates to a cell adhesion substrate for use in a method of regenerating a tissue in a subject, which method comprises transferring cells derived from a subject and caused to adhere to the cell surface adhesion substrate onto a biological tissue of the substrate in a patterned state and then growing the cells wherein the cell adhesion substrate comprises cell array substrate having a cell adhesiveness variation pattern that comprises regions having good cell adhesiveness and regions having inhibited cell adhesiveness patterned on a substrate, wherein cells adhere to the regions having good cell adhesiveness in the cell adhesiveness variation pattern, and wherein

the regions having good cell adhesiveness in the cell adhesiveness variation pattern have water contact angles between 10° and 40°.

The present invention further relates to said cell adhesion substrate for use in a method of treatment, said method comprising transferring vascular endothelial cells cause to adhere to the cell adhesion substrate onto the surface of an organ in a patterned state and then transferring the organ into a subject.

In an embodiment of the cell adhesion substrate for use of the present invention, the cell adhesiveness variation pattern is formed of a cell adhesiveness variation layer that comprises a cell adhesiveness variation material whose cell adhesiveness is varied by the action of a photocatalyst along with energy irradiation.

In an embodiment of the cell adhesion substrate for use of the present invention, the cell adhesiveness variation layer is a photocatalyst-comprising cell adhesiveness variation layer that comprises a photocatalyst and the cell adhesiveness variation material.

In an embodiment of the cell adhesion substrate for use of the present invention, the cell adhesiveness variation layer comprises a photocatalyst-comprising photocatalyst treatment layer and a cell adhesiveness variation material layer that comprises the cell adhesiveness variation material formed on the photocatalyst treatment layer.

In an embodiment of the cell adhesion substrate for use of the present invention, the above cell adhesiveness variation pattern is formed by arranging the cell adhesiveness variation layer that comprises the cell adhesiveness variation material and the photocatalyst-comprising layer so that the layers face each other, and then carrying out energy irradiation.

According to the present invention, cells can be arrayed and then cultured in a fine pattern using a convenient method without damaging the cells.

The cell culture method of the present invention is characterized by causing cells to adhere to the surface of a cell array substrate, transferring the adhered cells to a cell culture substrate in a patterned state, and then culturing the transferred cells. Such cell array substrate, a production method thereof, cell adhesion substrate upon which cells have adhered to the regions having good cell adhesiveness of the cell array substrate, transfer of the cells to the cell culture substrate, and culture of the transferred cells will be each explained as follows.

### I. Cell array substrate

First, the cell array substrate of the present invention will be explained in detail. The cell array substrate of the present invention is characterized in having a cell adhesiveness variation pattern that comprises regions having good cell adhesiveness and regions having inhibited cell adhesiveness patterned on a substrate.

"Cell adhesiveness" means strength for the adhesion of cells; that is, the degree of ease with which cells adhere. "Regions having good cell adhesiveness" means regions wherein cell adhesiveness is good. "Regions having inhibited cell adhesiveness" means regions wherein cell adhesiveness is poor. Accordingly, when cells are inoculated on such cell array substrate having a cell adhesiveness variation pattern, cells adhere to the regions having good cell adhesiveness, but no cells adhere to the regions having inhibited cell adhesiveness. Hence, cells are arrayed in a pattern on the surface of the cell array substrate.

Cell adhesiveness can differ depending on cells that are caused to adhere. Hence, "good cell adhesiveness" means that cell adhesiveness for a specific type of cell is good. Therefore, a plurality of regions having good cell adhesiveness are present corresponding to a plurality of types of cells on a cell array substrate. Specifically, regions having good cell adhesiveness, which vary in cell adhesiveness (2 or more different levels) may be present on the cell array substrate.

An example of the cell adhesiveness variation pattern is formed by forming a cell adhesiveness variation layer that comprises a cell adhesiveness variation material whose cell adhesiveness is varied along with energy irradiation on a substrate, varying cell adhesiveness through energy irradiation on specific regions, and then forming a pattern wherein regions differ in cell adhesiveness. Examples of such material whose cell adhesiveness is varied include both a material whose cell adhesiveness is acquired or increased along with energy irradiation and a material whose cell adhesiveness is decreased or disappears along with energy irradiation.

A substrate used for the cell array substrate of the present invention is not particularly limited, as long as it is formed of a material capable of forming a cell adhesiveness variation pattern on its surface. Specific examples of such substrate include inorganic materials such as metal, glass, and silicon, and organic materials represented by plastic (e.g., polyester resin, polyethylene resin, polypropylene resin, ABS resin, nylon, acrylic resin, fluorocarbon resin, polycarbonate resin, polyurethane resin, methylpentene resin, phenol resin, melamine resin, epoxy resin, and vinyl chloride resin). The shape of such material is also not limited. Examples of such shape include a flat plate, a flat membrane, a film, and a porous membrane. When a film is used, the thickness thereof is not particularly limited and is generally between 0.1 µm and 1000 µm, preferably between 1 µm and 500 µm, and more preferably between 20 µm and 200 µm.

The cell adhesiveness variation material and the cell adhesiveness variation layer will be explained in an embodiment using a photocatalyst.

Another example included herein is a cell adhesiveness variation pattern that is formed of a non-cell-adhesion layer that comprises a non-cell-adhesive material lacking cell adhesiveness and a cell adhesion layer that is formed on the non-cell-adhesion layer and comprises a cell adhesive material having cell adhesiveness. Here, the cell adhesion layer is decomposed and then disappears along with energy irradiation to cause the non-cell-adhesion layer to be exposed, so that regions differing in cell adhesiveness are formed. Similarly, another example included herein is a cell adhesiveness variation pattern that is formed of a cell adhesion layer and a non-cell-adhesion layer formed on the cell adhesion layer, wherein the non-cell-adhesion layer is decomposed and then disappears along with energy irradiation to cause the cell adhesion layer to be exposed, so that regions differing in cell adhesiveness are formed.

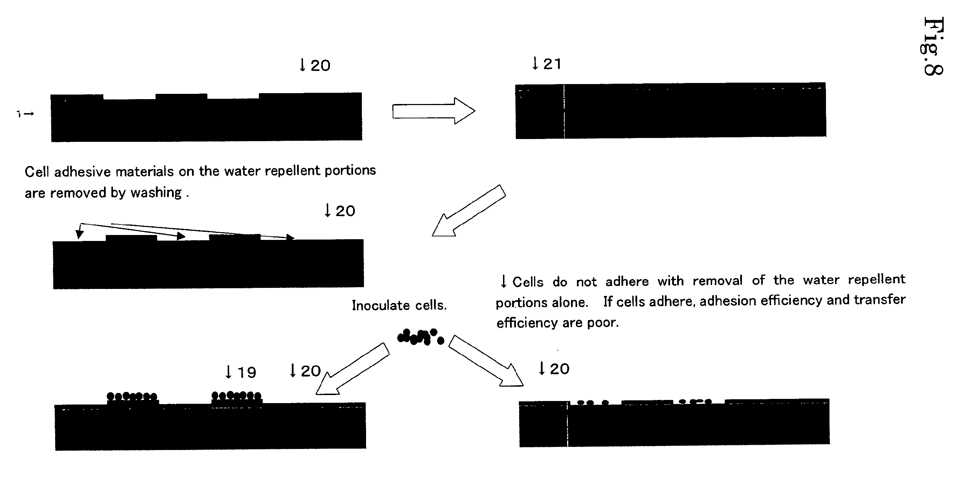

Examples of such cell adhesive material include extracellular matrices such as various types of collagen, fibronectin, laminin, vitronectin, and cadherin, and RGD peptide. Another example of the same is a polyolefin resin wherein a carbonyl group or a carboxyl group has been introduced by a technique such as corona treatment, ion beam irradiation treatment, or electron beam irradiation treatment in order to impart cell adhesiveness. Examples of such non-cell-adhesive material include fluorine materials such as polytetrafluoroethylene (PTFE), polyimide, and phospholipid. Moreover, through the use of a method such as an inkjet method, a cell adhesive material may be put on a non-cell-adhesion layer to form a pattern or a non-cell-adhesive material may be put on a cell adhesion layer to form a pattern. Alternatively, a cell adhesiveness variation pattern that comprises regions where a cell adhesive material is present (regions having good cell adhesiveness) and regions where a cell adhesive material is absent (regions having inhibited cell adhesiveness) can also be formed by: forming a layer that comprises an affinity variation material whose affinity for a cell adhesive material is varied along with energy irradiation on a substrate; forming a pattern through energy irradiation that comprises regions having affinity for the cell adhesive material and regions lacking such affinity; introducing a solution that contains the cell adhesive material; and then washing. In such embodiment, a pattern can be formed using a cell adhesive material that cannot be directly patterned on a substrate. For example, as shown in Fig. 8, a pattern that comprises regions (20) comprising a layer that comprises a water repellent material and regions lacking such material is formed on a hydrophilic substrate (1) such as glass. A hydrophilic cell adhesive material (21) that is hardly adsorbed to the water repellent material is introduced thereto. The substrate is then washed. Accordingly, regions wherein the hydrophilic cell adhesive material is present (regions having good cell adhesiveness) and regions wherein the water repellent material is present (regions having inhibited cell adhesiveness) form a pattern. An extracellular matrix such as collagen can be used as such hydrophilic cell adhesive material to be used in this case.

In the present invention, cells arrayed in a pattern on a cell array substrate are transferred to a cell culture substrate. Hence, the cell adhesiveness of the above regions having good cell adhesiveness is preferably at a proper strength. With such proper adhesion strength, it becomes possible to transfer the cells to a cell culture substrate, while forming a cell pattern by adhering cells only to specific regions. Therefore, it is preferable that the cell adhesiveness of regions having good cell adhesiveness on a cell array substrate is higher than those of regions having inhibited cell adhesiveness, but lower than that of the surface of a cell culture substrate.

Such cell adhesiveness can be evaluated using a water contact angle on the surface. Regions having good cell adhesiveness of the cell adhesiveness variation pattern in the present invention each have a water contact angle between 10° and 40°. When cells are caused to adhere to a cell array substrate and then transferred to a cell culture substrate with a water contact angle within such range, the cells can be caused to adhere to a cell array substrate in the form of a monolayer. And then, the cells can be easily transferred to a cell culture substrate because of weak adhesiveness to the cell array substrate. "Contact angle" means an angle formed by the surface of a liquid and the surface of a solid where the free surface of the liquid at rest comes into contact with the wall of the solid (angle measured within the liquid).

The term "water contact angle" used herein means a value measured using a static contact angle measurement method. The above water contact angle is generally observed by adding minute waterdrops dropwise to the surface of a material under atmospheric pressure using an instrument such as a syringe and then observing the angle formed by the liquid/vapor interface (at the droplet end) and the surface of a solid using a magnifying glass or the like.

There is no particular limitation of a means for forming the above cell adhesiveness variation pattern that comprises regions having good cell adhesiveness and regions having inhibited cell adhesiveness arranged in a pattern. Examples of such means include various printing methods such as a gravire printing method, a screen printing method, an offset printing method, a flexographic printing method, and a contact printing method, a method using various lithographic methods, a method based on an inkjet method, and three-dimensional modeling such as carving fine grooves. In the present invention, a lithographic method using a photocatalyst is preferable. Specifically, in such method, a cell adhesiveness variation material (whose cell adhesiveness is varied by the action of a photocatalyst along with energy irradiation) and the photocatalyst are used and energy irradiation that is carried out according to a required pattern, so as to form a required cell adhesiveness variation pattern. In such embodiment, a high-definition pattern can be formed with convenient steps without using any treatment solution that adversely affects cells. Moreover, such embodiment does not require any modification of a cell adhesiveness variation material. Thus, options for material selection can be expanded. Furthermore, a biological cell adhesiveness variation material that exerts specific adhesiveness described later can also be used without any problems.

A pattern to be formed is not particularly limited, as long as it is a two-dimensional pattern. Such pattern can be selected depending on types of cell to be arrayed on a cell array substrate, transferred, and then cultured, types of tissues to be formed, and the like. For example, a linear, tree-shaped, mesh, grid, circular, or quadrille pattern, a pattern wherein the inside of a figure (e.g., circle and tetragram) consists of regions having good cell adhesiveness or regions having inhibited cell adhesiveness, or the like can be formed. When a tissue is formed through the culture of vascular endothelial cells or nerve cells, it is preferable to form a pattern so that cells adhere to form a linear, tree-shaped, mesh, or grid pattern. When the patterned cells are transferred to and cultured on a cell culture substrate, in the case of vascular endothelial cells, tissue formation is promoted and angiogenesis is thus promoted because the cells are arrayed in a linear, tree-shaped, mesh, or grid pattern. When such linear, tree-shaped, mesh, or grid pattern, is formed, the line width in the pattern is generally between 20 µm and 200 µm and preferably between 50 µm and 100 µm. In particular, when capillary vessels are formed by arranging and culturing vascular endothelial cells in a line-shaped pattern, it is preferable that a cell adhesiveness variation pattern is formed where linear regions having good cell adhesiveness and spaces comprised of regions having inhibited cell adhesiveness are arranged alternately, so as to cause the vascular endothelial cells to adhere to form a linear pattern. In such embodiment, a pattern is preferably formed wherein cells are caused to adhere so that a line width can contain 1 to 10 cells and preferably 1 to 5 cells. Specifically, the line width of a region having good cell adhesiveness is generally between 20 µm and 200 µm, and preferably between 50 µm and 80 µm. The space widths between such lines (the spaces comprised of regions having inhibited cell adhesiveness) are each generally between 300 µm and 1000 µm and preferably between 400 µm and 800 µm. With a line width determined within the above numerical range, vascular endothelial cells can efficiently form a tubular tissue. Through the formation of such cell adhesiveness variation pattern, vascular endothelial cells caused to adhere and then transferred in a linear pattern efficiently form a tissue; that is, a linear capillary vessel. When a cell pattern where a plurality of lines are arranged without crossing each other is formed, the space widths between the lines on which cells are adhered are each set to be equal to or above a specific value as described above. Accordingly, the cells can be prevented from extending pseudopodia between the lines at the time of their tissue formation, which would distort the lines.

Furthermore, when a capillary vessel is formed by arranging and culturing vascular endothelial cells in a grid pattern, it is preferable to arrange alternately linear regions having good cell adhesiveness and spaces comprised of regions having inhibited cell adhesiveness as described above, to form a cell adhesiveness variation pattern where the linear regions having good cell adhesiveness crossing the above lines are further arranged, and then to cause vascular endothelial cells to adhere to form a grid pattern. In this case, the width of lines crossing each other is similar to that described above. Each space width between additional lines is generally between 0.03 cm and 5 cm and preferably between 0.04 cm and 3 cm.

Examples of the above cell array substrate prepared by a lithographic method using a photocatalyst include the following three embodiments. Each of these embodiments will be described as follows.

### A. 1^{st} Embodiment

A 1^{st} embodiment of the cell array substrate of the present invention is a cell array substrate comprising a cell adhesiveness variation layer that is formed on a substrate and comprises a cell adhesiveness variation material whose cell adhesiveness is varied by the action of a photocatalyst along with energy irradiation, wherein the above cell adhesiveness variation layer forms a cell adhesiveness variation pattern with variations in cell adhesiveness characterized in that the above cell adhesiveness variation layer is a photocatalyst-comprising cell adhesiveness variation layer that comprises the photocatalyst and the above cell adhesiveness variation material.

In this embodiment, the cell adhesiveness variation layer is a photocatalyst-comprising cell adhesiveness variation layer that comprises the photocatalyst and the above cell adhesiveness variation material. Thus, when energy irradiation is carried out, the cell adhesiveness of the cell adhesiveness variation material is varied by the action of the photocatalyst within the photocatalyst-comprising cell adhesiveness variation layer. Hence, a cell adhesiveness variation pattern that comprises portions subjected to energy irradiation and portions not subjected to energy irradiation, where the portions differ in terms of cell adhesiveness, can be formed.

Members used in such cell array substrate in this embodiment will be each described as follows.

### 1. Photocatalyst-comprising cell adhesiveness variation layer

This embodiment is characterized in that a photocatalyst-comprising cell adhesiveness variation layer is formed on a substrate. The photocatalyst-comprising cell adhesiveness variation layer comprises at least a photocatalyst and a cell adhesiveness variation material.

### (1) Cell adhesiveness variation material

A cell adhesiveness variation material used in this embodiment is not particularly limited, as long as it is a material whose cell adhesiveness is varied by the action of a photocatalyst along with energy irradiation. Examples of such material whose cell adhesiveness is varied include both a material that acquires or increases its cell adhesiveness by the action of a photocatalyst along with energy irradiation and a material whose cell adhesiveness decreases or disappears due to the action of a photocatalyst along with energy irradiation.

There are two major embodiments of such cell adhesiveness variation material, which differ in an aspect to control cell adhesiveness. One embodiment is a physicochemical cell adhesiveness variation material that adheres to cells due to its physicochemical property and the other embodiment is a biological cell adhesiveness variation material that adheres to cells due to its biological property.

### a. Physicochemical cell adhesiveness variation material

Examples of a physicochemical factor for causing cells to adhere to the surface include a factor relating to surface free energy, a factor relating to hydrophobic interaction, and the like.

A preferable physicochemical cell adhesive material having physicochemical cell adhesiveness due to the presence of such factor possesses binding energy that is sufficiently high so that the main backbone is not decomposed by the action of a photocatalyst and also has an organic substituent that is decomposed by the action of a photocatalyst. Examples of such material include (1) organopolysiloxane that is obtained through hydrolysis and polycondensation of such as chloro- or alkoxysilane using a sol-gel reaction or the like so as to exert high strength and (2) organopolysiloxane that is obtained through crosslinking of reactive silicones.

In the case of (1) above, a preferable organopolysiloxane is 1 or 2 or more types of hydrolysis condensate or cohydrolysis condensate of a silicon compound that is represented by general formula:

Y_{b}SiX₍₄₋ₙ₎

(where Y indicates an alkyl group, a fluoroalkyl group, a vinyl group, an amino group, a phenyl group, or an epoxy group; X indicates an alkoxyl group, an acetyl group, or halogen; and "n" is an integer between 0 and 3). In addition, the carbon number of a group indicated with Y is preferably within the range between 1 and 20. Furthermore, an alkoxy group indicated with X is preferably a methoxy group, an ethoxy group, a propoxy group, or a butoxy group.

Furthermore, as an organic group, polysiloxane comprising a fluoroalkyl group can be particularly preferably used. Specific examples of such polysiloxane include 1 or 2 or more types of hydrolysis condensate and cohydrolysis condensate of the following fluoroalkyl silane. Such polysiloxane generally known as a fluorine silane coupling agent can be used. Examples are:
CF₃(CF₂)₃CH₂CH₂Si(OCH₃)₃;
CF₃(CF₂)₅CH₂CH₂Si(OCH₃)₃;
CF₃(CF₂)₇CH₂CH₂Si(OCH₃)₃;
CF₃(CF₂)₉CH₂CH₂Si(OCH₃)₃;
(CF₃)₂CF(CF₂)₄CH₂CH₂Si(OCH₃)₃;
(CF₃)₂CF(CF₂)₆CH₂CH₂Si(OCH₃)₃;
(CF₃)₂CF(CF₂)₈CH₂CH₂Si(OCH₃)₃;
CF₃(C₆H₄)C₂H₄Si(OCH₃)₃;
CF₃(CF₂)₃(C₆H₄)C₂H₄Si(OCH₃)₃;
CF₃(CF₂)₅(C₆H₄)C₂H₄Si(OCH₃)₃;
CF₃(CF₂)₇(C₆H₄)C₂H₄Si(OCH₃)₃;
CF₃(CF₂)₃CH₂CH₂SiCH₃(OCH₃)₂;
CF₃(CF₂)₅CH₂CH₂SiCH₃(OCH₃)₂;
CF₃(CF₂)₇CH₂CH₂SiCH₃(OCH₃)₂;
CF₃(CF₂)₉CH₂CH₂SiCH₃(OCH₃)₂;
(CF₃)₂CF(CF₂)₄CH₂CH₂SiCH₃(OCH₃)₂;
(CF₃)₂CF(CF₂)₆CH₂CH₂SiCH₃(OCH₃)₂;
(CF₃)₂CF(CF₂)₈CH₂CH₂SiCH₃(OCH₃)₂;
CF₃(C₆H₄)C₂H₄SiCH₃(OCH₃)₂;
CF₃(CF₂)₃(C₆H₄)C₂H₄SiCH₃(OCH₃)₂;
CF₃(CF₂)₅(C₆H₄)C₂H₄SiCH₃(OCH₃)₂;
CF₃(CF₂)₇(C₆H₄)C₂H₄SiCH₃(OCH₃)₂;
CF₃(CF₂)₃CH₂CH₂Si(OCH₂CH₃)₃;
CF₃(CF₂)sCH₂CH₂Si(OCH₂CH₃)₃;
CF₃(CF₂)₇CH₂CH₂Si(OCH₂CH₃)₃;
CF₃(CF₂)₉CH₂CH₂Si(OCH₂CH₃)₃; and
CF₃(CF₂)₇SO₂N(C₂H₅)C₂H₄CH₂Si(OCH₃)₃.

Through the use of the above polysiloxane comprising a fluoroalkyl group as a physicochemical cell adhesive material, a portion not subjected to energy irradiation of a photocatalyst-comprising cell adhesiveness variation layer will form a surface lacking cell adhesiveness because of the presence of a portion having fluorine on the surface. On the other hand, a portion subjected to energy irradiation will form a surface having cell adhesiveness because of the removal of fluorine and the like and the resulting presence of a portion having an OH group and the like on the surface. Therefore, regions can be patterned so that portions subjected to energy irradiation and portions not subjected to energy irradiation differ in terms of cell adhesiveness.

Moreover, an example of reactive silicone in (2) above is a compound having a backbone that is represented by the following general formula.

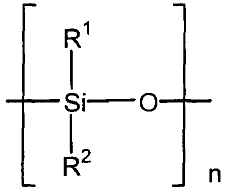

In the above formula, "n" is an integer of 2 or greater. R¹ and R² each indicate a C₁₋₁₀ substituted or unsubstituted alkyl, alkenyl, or aryl group. Examples of a substituent include halogen and cyano. Specific examples of R¹ and R² include methyl, ethyl, propyl, vinyl, phenyl, phenyl halide, cyano methyl, cyano ethyl, and cyano propyl. Vinyl, phenyl, or phenyl halide preferably constitutes 40% or less (in molar ratio) of the whole. Furthermore, a compound wherein R¹ and R² are each a methyl group is preferable because this results in the lowest surface energy. In addition, a methyl group constitutes preferably 60% (in molar ratio) or more of the whole. Furthermore, a chain terminus or a side chain has at least 1 or more reactive groups such as a hydroxyl group in a molecular chain.

Moreover, an organosilicone compound that does not undergo a crosslinking reaction and thus is stable, such as dimethyl polysiloxane, may also be separately mixed with the above organopolysiloxane.

Furthermore, an example of a physicochemical cell adhesive material (of a decomposable substance type) is a surfactant that is decomposed by the action of a photocatalyst and that has a function (exerted by decomposition) of varying the polarity of the surface of a photocatalyst-comprising polarity variation layer. Specific examples of such surfactant include hydrocarbon non-ionic surfactants (e.g., NIKKOL BL, BC, BO, and BB series produced by Nikko Chemicals Co., Ltd.) and fluorine or silicone non-ionic surfactants (e.g., ZONYL FSN and FSO produced by DuPont Kabushiki Kaisha, Surflon S-141 and 145 produced by ASAHI GLASS CO., LTD., Megafac F-141 and 144 produced by DAINIPPON INK AND CHEMICALS, INCORPORATED, FTERGENT F-200 and F251 produced by NEOS COMPANY LIMITED, Unidine DS-401 and 402 produced by DAIKIN INDUSTRIES, LTD., and Fluorad FC-170 and 176 produced by 3M (Minnesota Mining and Manufacturing Company). Moreover, a cationic surfactant, an anionic surfactant, or an amphoteric surfactant can also be used.

In addition, when a physicochemical cell adhesive material of a decomposable substance type is used as a material, generally preferably a binder component is separately used. Such binder component that is used in this case is not particularly limited, as long as it possesses binding energy that is sufficiently high so that the main backbone is not decomposed by the action of the above photocatalyst. Specific examples of such component include polysiloxane having no organic substituent and polysiloxane having little organic substituents. They can also be obtained by hydrolysis and polycondensation of tetramethoxysilane, tetraethoxysilane, or the like.

In addition, in this embodiment, a physicochemical cell adhesive material of such binder type and a physicochemical cell adhesive material of such decomposable substance type may be used together.

Another example is a physicochemical cell adhesiveness variation material whose cell adhesiveness is varied through the control of electrostatic interaction. In the case of such material, positively charged functional groups (contained in such material) are decomposed by the action of a photocatalyst along with energy irradiation. The amount of positive charge existing on the surface is then varied so as to vary adhesiveness between the surface and cells. Thus, a cell adhesiveness variation pattern is formed. An example of such material is poly L lysine.

### b. Biological cell adhesiveness variation material

Examples of a biological factor for causing cells to adhere to the surface include a material that can have a property of adhering to many cell types and a material that has a property of adhering to only a specific cell type. The former material is a collagen I type material, for example. The latter material is poly(N-p-vinylbenzyl-[O-β-D-galactopyranosyl-(1→4)-D-gluconamide])(hereinafter, PVLA) that causes selective adhesion of hepatic parenchymal cells, for example. In the case of PVLA, it is inferred that selective and specific material-to-cell adhesion occurs, because PVLA has a galactose group that is specifically recognized by hepatic parenchymal cells in its structure.

When such material is mixed with a photocatalyst and then used for a photocatalyst-comprising cell adhesiveness variation layer, the following type of usage is possible. Collagen I type material is solubilized by enzyme treatment. The thus solubilized collagen I is mixed with a photocatalyst such as a TiO₂ particle that has been previously subjected to calcination treatment and grinding treatment, thereby preparing a material for a photocatalyst-comprising cell adhesiveness variation layer. Next, the material for the photocatalyst-comprising cell adhesiveness variation layer is applied to a substrate, thereby forming a photocatalyst-comprising cell adhesiveness variation layer. When the photocatalyst-comprising cell adhesiveness variation layer is irradiated with a small amount of energy, a cell adhesive peptide structure in a side chain of collagen is partially disrupted. Thus, cell adhesiveness can be decreased. Furthermore, such cell adhesive peptide structure can be gradually caused to disappear by increasing the amount of energy irradiation. Furthermore, cell adhesiveness can be further decreased.

Furthermore, an excessive amount of energy irradiation can lead to disruption of the main chain structure of collagen and complete loss of the cell adhesiveness.

### (2) Photocatalyst

Examples of a photocatalyst that is used in this embodiment include those known as optical semiconductors such as titanium dioxide (TiO₂), zinc oxide (ZnO), tin oxide (SnO₂), strontium titanate (SrTiO₃), tungsten oxide (WO₃), bismuth oxide (Bi₂O₃), and iron oxide (Fe₂O₃). 1 or 2 or more types of optical semiconductor can be selected from the above examples, mixed, and then used.

In this embodiment, titanium dioxide is particularly preferably used because it possesses high band gap energy, is chemically stable, is free from toxicity, and can be easily obtained. There exist anatase-type and rutile-type titanium dioxide, and both can be used in this embodiment. The anatase-type titanium dioxide is preferable. The anatase type titanium dioxide has an excitation wavelength of 380 nm or less.

Examples of such anatase-type titanium dioxide include anatase-type titaniasol of hydrochloric acid deflocculation type (STS-02 (average particle size of 7 nm) produced by ISHIHARA SANGYO KAISHA, LTD. and ST-K01 produced by ISHIHARA SANGYO KAISHA, LTD.) and anatase-type titaniasol of nitric acid deflocculation type (TA-15 (average particle size of 12 nm) produced by NISSAN CHEMICAL INDUSTRIES, LTD.).

A smaller photocatalyst particle size is preferable, because photocatalyst reactions take place more effectively with smaller particle size. The average particle diameter is preferably 50 nm or less. It is particularly preferable to use a photocatalyst with an average diameter of 20 nm or less.

The content of a photocatalyst in a photocatalyst-comprising cell adhesiveness variation layer that is used in this embodiment can be determined to be within a range between 5 wt.% and 60 wt.%, and preferably between 20 wt.% and 40 wt.%.

### 2. Substrate

A substrate that is used as the cell array substrate of the present invention is not particularly limited, as long as it is formed of a material with which a photocatalyst-comprising cell adhesiveness variation layer can be formed on the surface. Any form can be selected for such substrate, as long as surface treatment can be carried out through exposure treatment. Specific examples of such material include inorganic materials such as metal, glass, and silicon and organic materials represented by plastic. The shape of such substrate is also not limited. Examples of such shape include a flat plate, a flat membrane, a film, and a porous membrane.

### 3. Cell adhesiveness variation pattern

In this embodiment, the above-described photocatalyst-comprising cell adhesiveness variation layer is formed on the above substrate, and then the substrate is subjected to energy irradiation in a pattern. Thus, a cell adhesiveness variation pattern with variation in cell adhesiveness is formed.

Such cell adhesiveness variation pattern is generally formed of regions having good cell adhesiveness (with good cell adhesiveness) and regions having inhibited cell adhesiveness (with poor cell adhesiveness). Through adhesion of cells to the regions having good cell adhesiveness, the cells can be adhered in a high-definition pattern. Such regions having good cell adhesiveness and such regions having inhibited cell adhesiveness are determined depending on the type of a cell adhesiveness variation material that is used herein.

For example, a cell adhesiveness variation material may be a physicochemical cell adhesiveness variation material that causes variation in cell adhesiveness by varying the surface free energy. In such case, the surface free energy that is within a predetermined range tends to result in good cell adhesiveness, but the surface free energy that is out of such range tends to result in decreased cell adhesiveness. A known example of variation in cell adhesiveness due to surface free energy is provided by the experimental results shown in the lower part of page 109, Frontiers of Biomaterials, under the general editorship of Yoshihito Ikada, CMC Publishing CO., LTD.

Cell adhesiveness can also be determined depending not only on the surface free energy of the above material, but also on the combination of a cell type and a material type that are caused to come into contact.

Here, such cell adhesiveness variation pattern is a pattern comprising the above regions having good cell adhesiveness and the above regions having inhibited cell adhesiveness. Depending on its application, it may be a cell adhesiveness variation pattern comprising regions where surface cell adhesiveness is varied by at least 3 different levels.

This is because, in a case where a photocatalyst-comprising cell adhesiveness variation layer comprising a biological cell adhesiveness variation material is used or in a case where it has not yet been confirmed if cell adhesiveness is good, or the like, it may be advantageous in terms of ability of finding optimum states for adhesiveness by successively causing changes to the surface states of a photocatalyst-comprising cell adhesiveness variation layer.

As described above, in the present invention, 3 or more different levels of cell adhesiveness include a state where cell adhesiveness is successively varied. The appropriate level of cell adhesiveness is appropriately selected depending on each circumstance and then determined.

Regions having such multiple different levels of adhesiveness can be formed by varying the amount of energy irradiation to a photocatalyst-comprising cell adhesiveness variation layer. Specifically, an example is a method using half-tone photomasks varying in transmittance.

Furthermore, a cell adhesiveness variation pattern that can be used in this embodiment uses a difference in photocatalyst activity between a portion subjected to energy irradiation and a portion not subjected to energy irradiation. Specifically, for example, a biological cell adhesiveness variation material that has been introduced as a decomposable substance into a photocatalyst-comprising cell adhesiveness variation layer is used. When the surface of such photocatalyst-comprising cell adhesiveness variation layer is irradiated with energy in a pattern, the biological cell adhesiveness variation material that has exuded on the surface of the irradiated portion is decomposed and the biological cell adhesiveness variation material of the unirradiated portion remains. Hence, when such biological cell adhesiveness variation material has good adhesiveness to a specific cell type or has good adhesiveness to many cell types, such unirradiated portions become to be regions having good cell adhesiveness. The irradiated portions become to be regions where a biological cell adhesiveness variation material having good adhesiveness to a cell is absent. Furthermore, the irradiated portions also become to be regions where a photocatalyst having activated sterility has been exposed as a result of energy irradiation. Therefore, when an energy-irradiated portion results in regions having inhibited cell adhesiveness, particularly when culture is carried out using the cell array substrate in this embodiment for a predetermined time period, such regions are advantageous in terms of not causing problems such as a thicker pattern width.

### B. 2^{nd} embodiment

A 2^{nd} embodiment of the cell array substrate of the present invention is a cell array substrate comprising a substrate and a cell adhesiveness variation layer that is formed on the substrate and comprises a cell adhesiveness variation material whose cell adhesiveness is varied by the action of a photocatalyst along with energy irradiation, wherein the above cell adhesiveness variation layer forms a cell adhesiveness variation pattern with variation in cell adhesiveness characterized in that: the above cell adhesiveness variation layer comprises a photocatalyst-comprising photocatalyst treatment layer and a cell adhesiveness variation material layer that is formed on the above photocatalyst treatment layer and comprises the above cell adhesiveness variation material.

In this embodiment, such cell adhesiveness variation layer comprises a photocatalyst treatment layer formed on a substrate and a cell adhesiveness variation material layer formed on the photocatalyst treatment layer. Thus, when energy irradiation is carried out, the cell adhesiveness of the cell adhesiveness variation material within the cell adhesiveness variation material layer is varied by the action of the photocatalyst within the photocatalyst treatment layer. Hence, a cell adhesiveness variation pattern comprising portions subjected to energy irradiation and portions not subjected to energy irradiation, where the portions differ in terms of cell adhesiveness, can be formed.

Members used in such cell array substrate in this embodiment will be separately described as follows.

### 1. Cell adhesiveness variation material layer

In the cell array substrate of this embodiment, a cell adhesiveness variation material layer is formed on a photocatalyst treatment layer that is formed on the substrate. As such cell adhesiveness variation material layer, a layer that is formed with the use of a cell adhesiveness variation material explained in the above 1^{st} embodiment can be used. A cell adhesiveness variation material layer prepared with the use of a physicochemical cell adhesiveness variation material and a cell adhesiveness variation material layer prepared with the use of a biological cell adhesiveness variation material will be separately explained as follows.

### (1) Use of physicochemical cell adhesiveness variation material

In this embodiment, a cell adhesiveness variation material layer formed of a physicochemical cell adhesiveness variation material may be prepared as a layer prepared with the use of a material similar to that explained in the above 1^{st} embodiment. When such material is used, the thus prepared layer is similar to the above layer except for the presence or the absence of a photocatalyst. In addition, in this embodiment, a cell adhesiveness variation material layer is not principally required to comprise a photocatalyst therewithin, but may comprise a photocatalyst in a small amount in view of sensitivity or the like.

Furthermore, in this embodiment, a cell adhesiveness variation material layer is formed as a layer to be decomposed and removed (that is, the layer is decomposed and removed by the action of a photocatalyst) on a photocatalyst treatment layer. Regions wherein the cell adhesiveness variation material layer has been decomposed by the action of the photocatalyst along with energy irradiation (that is, regions wherein the photocatalyst treatment layer has been exposed) and regions wherein the cell adhesiveness variation material layer has remained are then formed. Thus, a cell adhesiveness variation pattern is formed. Such type of cell adhesiveness variation material layer having the thus formed pattern can be used.

Specifically, when cell adhesiveness is controlled with surface free energy, a physicochemical cell adhesiveness variation material whose surface free energy is appropriate for cell adhesiveness is used. Such material is applied to the whole surface, thereby forming a cell adhesiveness variation material layer. Subsequently, patterned energy irradiation is carried out according to a pattern, so as to form a pattern comprising regions of presence of and of absence of the cell adhesiveness variation material layer. Thus, a cell adhesiveness variation pattern is formed.

Examples of such physicochemical cell adhesiveness variation material layer that is used as a layer to be decomposed and removed and can be used for controlling cell adhesiveness with surface free energy include regenerated cellulose and nylon 11.

Furthermore, when cell adhesiveness is controlled with electrostatic interaction, a cell adhesiveness variation pattern can be formed using a positively charged physicochemical cell adhesiveness variation material and by a method similar to the above method.

Examples of a material that is used for such physicochemical cell adhesiveness variation material layer used as a layer to be decomposed and removed and can be used for controlling cell adhesiveness with electrostatic interaction include polyamine graft poly (2-hydroxymethylmethacrylate)(HA-x) and the like.

These resins are dissolved in a solvent and a film can be formed by a general film production method such as a spin coat method. Moreover, in the present invention, a defect-free film can be formed with the use of a functional thin film such as a self-organizing monomolecular film, Langmuir-Blodgett film, or an alternate adsorption film. Thus, it is preferable to use such film production method.

When a cell adhesiveness variation pattern is formed using a cell adhesiveness variation material layer as such layer to be decomposed and removed, regions subjected to decomposition and removal are regions within which cell culture is greatly inhibited, because in such regions a photocatalyst treatment layer is exposed.. Hence, a cell array substrate that is obtained by such method is advantageous in that it can maintain a high-definition pattern even after keeping cells for a long time.

### (2) Use of biological cell adhesiveness variation material

In this embodiment, a material similar to that explained in the 1^{st} embodiment can be used for a cell adhesiveness variation material layer that is formed of a biological cell adhesiveness variation material. An example of such material is collagen I type.

### 2. Photocatalyst treatment layer

Next, a photocatalyst treatment layer that is used in the present invention will be explained. Such photocatalyst treatment layer used in the present invention is not particularly limited, as long as it is constituted in such a manner that the cell adhesiveness of a cell adhesiveness variation material layer (which is formed on a photocatalyst treatment layer) is varied by a photocatalyst in the photocatalyst treatment layer. Such photocatalyst treatment layer may be a layer composed of a photocatalyst and a binder or a layer prepared by a film production method using a photocatalyst alone. Furthermore, the surface may particularly be lyophilic or lyophobic. A lyophilic surface is preferable in terms of convenience of forming a cell adhesiveness variation material layer and the like on the photocatalyst treatment layer.

In such photocatalyst treatment layer, the action mechanism of a photocatalyst represented by titanium oxide that is described later is not always clear. It is thought that a direct reaction between a carrier generated by light irradiation and a neighboring compound or active oxygen species generated in the presence of oxygen and water causes a change in the chemical structure of organic matters. In the present invention, it is considered that such carrier acts on a compound in a cell adhesiveness variation material layer formed on a photocatalyst treatment layer. Examples of such photocatalyst are similar to those described in detail in the 1^{st} embodiment.

The photocatalyst treatment layer in this embodiment may be a layer formed of a photocatalyst alone as described above or a layer formed by mixing it with a binder.

A photocatalyst treatment layer consisting of a photocatalyst alone is advantageous in terms of cost, because its efficiency of causing variation in the cell adhesiveness of a cell adhesiveness variation material layer is improved and a treatment time is reduced. On the other hand, a photocatalyst treatment layer consisting of a photocatalyst and a binder is advantageous in that a photocatalyst treatment layer can be easily formed.

Examples of a method for forming a photocatalyst treatment layer consisting of a photocatalyst alone include a sputtering method, a CVD method, and a vacuum film production method such as a vacuum deposition method. Formation of a photocatalyst treatment layer by the vacuum film production method enables preparation of a photocatalyst treatment layer formed of a uniform film and comprising a photocatalyst alone. Thus, the properties of a cell adhesiveness variation material layer can be uniformly varied. Furthermore, since such layer consists of a photocatalyst alone, it becomes possible to vary the cell adhesiveness of a cell adhesiveness variation layer more efficiently than in the case of using a binder.

Moreover, another example of a method for forming a photocatalyst treatment layer consisting of a photocatalyst alone is, when a photocatalyst is titanium dioxide, a method that comprises forming amorphous titania on a substrate and then causing a phase change through calcination to obtain crystalline titania. Amorphous titania that is used herein can be obtained by hydrolysis or dehydration and condensation of inorganic salts of titanium, such as titanium tetrachloride and titanium sulfate, or hydrolysis, or dehydration and condensation of an organic titanium compound, such as tetraethoxy titanium, tetraisopropoxy titanium, tetra-n-propoxy titanium, tetrabutoxy titanium, and tetramethoxy titanium in the presence of acid. Subsequently, such titania is modified to result in anatase-type titania through calcination at a temperature between 400C° and 500°C or to result in rutile-type titania through calcination at a temperature between 600°C and 700°C.

Furthermore, when a binder is used, a preferable binder possesses binding energy that is sufficiently high so that the main backbone of the binder is not decomposed by the action of the above photocatalyst. Examples of such binder include the above organopolysiloxane and the like.

When organopolysiloxane is used as a binder as described above, the above photocatalyst treatment layer can be formed by dispersing a photocatalyst and organopolysiloxane that is the binder in a solvent together with another additive, if necessary, preparing an application solution, and then applying the application solution to a transparent substrate. A solvent that is used herein is preferably an alcohol-based organic solvent such as ethanol or isopropanol. Application can be carried out by a known application method such as a spin coating, a spray coating, a dip coating, a roll coating, or a bead coating method. When a UV-hardened type component is contained as a binder, a photocatalyst treatment layer can be formed through UV irradiation to carry out hardening treatment.

Furthermore, an amorphous silica precursor can be used as a binder. As such amorphous silica precursor, a silicon compound represented by general formula SiX₄, wherein X is halogen, a methoxy group, an ethoxy group, an acetyl group, or the like, silanol that is a hydrolysate thereof, or polysiloxane with an average molecular weight of 3000 or less is preferable.

Specific examples of such precursor include tetraethoxysilane, tetraisopropoxy silane, tetra-n-propoxy silane, tetrabutoxy silane, and tetramethoxysilane. Furthermore, in this case, a photocatalyst treatment layer can be formed by uniformly dispersing an amorphous silica precursor and photocatalyst particles in a non-aqueous solvent, subjecting the resultant to hydrolysis on a transparent substrate by water in air, so as to form silanol, and then carrying out dehydration, condensation, and polymerization at normal temperature. A process of dehydration, condensation, and polymerization of silanol at 100°C or higher results in an increased polymerization degree of silanol and thus can improve the strength of the film surface. Moreover, such binding agent can be used alone, or a mixture of 2 or more types of such binding agents can be used.

The content of a photocatalyst in a photocatalyst treatment layer when a binder is used can be determined within a range between 5 wt.% and 60 wt.%, and preferably between 20 wt.% and 40 wt.%. Furthermore, the thickness of a photocatalyst treatment layer is preferably within a range between 0.05 µm and 10 µm.

Furthermore, a photocatalyst treatment layer may comprise a surfactant in addition to the above photocatalyst and binder. Specific examples of such surfactant include hydrocarbon non-ionic surfactants (e.g., NIKKOL BL, BC, BO, and BB series produced by Nikko Chemicals Co., Ltd.), fluorine or silicone non-ionic surfactants (e.g., ZONYL FSN and FSO produced by DuPont Kabushiki Kaisha, Surflon S-141 and 145 produced by ASAHI GLASS CO., LTD., Megafac F-141 and 144 produced by DAINIPPON INK AND CHEMICALS, INCORPORATED, FTERGENT F-200 and F251 produced by NEOS COMPANY LIMITED, Unidine DS-401 and 402 produced by DAIKIN INDUSTRIES, LTD., and Fluorad FC-170 and 176 produced by 3M (Minnesota Mining and Manufacturing Company). Moreover, a cationic surfactant, an anionic surfactant, or an amphoteric surfactant can also be used.

Furthermore, a photocatalyst treatment layer can comprise, in addition to the above surfactant, an oligomer, a polymer, or the like such as polyvinyl alcohol, unsaturated polyester, acrylic resin, polyethylene, diallyl phthalate, ethylene propylene diene monomer, epoxy resin, phenol resin, polyurethane, melamine resin, polycarbonate, poly(vinyl chloride), polyamide, polyimide, styrene butadiene rubber, chloroprene-rubber, polypropylene, polybutylene, polystyrene, poly(vinyl acetate), polyester, polybudadiene, polybenzimidazole, polyacryl nitrile, epichlorohydrin, polysulfide, and polyisoprene.

### 3. Substrate

A substrate that is used in this embodiment is not particularly limited, as long as the above photocatalyst treatment layer can be formed. A substrate similar to that explained in the 1^{st} embodiment can be used.

### 4. Cell adhesiveness variation pattern

In this embodiment, a cell adhesiveness variation pattern is formed, wherein the cell adhesiveness of the surface of a cell adhesiveness variation material layer is varied by the action of a photocatalyst in a photocatalyst treatment layer as a result of energy irradiation carried out in a pattern on the above cell adhesiveness variation material layer.

### C. Third embodiment

A cell array substrate in this embodiment comprises a substrate and a cell adhesiveness variation layer that is formed on the above substrate and comprises a cell adhesiveness variation material whose cell adhesiveness is varied by the action of a photocatalyst along with energy irradiation, wherein the above cell adhesiveness variation layer forms a cell adhesiveness variation pattern with variation in cell adhesiveness, characterized in that: the above cell adhesiveness variation layer is a cell adhesiveness variation material layer comprising the above cell adhesiveness variation material; and the above adhesiveness variation pattern is formed by arranging a photocatalyst-comprising layer and the above cell adhesiveness variation material layer so that the layers face each other, and then carrying out energy irradiation from a predetermined direction.

In this embodiment, a cell adhesiveness variation layer is a cell adhesiveness variation material layer as described above; and the above adhesiveness variation pattern is formed by arranging a photocatalyst-comprising layer and the above cell adhesiveness variation material layer so that the layers face each other, and then carrying out energy irradiation from a predetermined direction. Thus, at the time of energy irradiation, the cell adhesiveness of the cell adhesiveness variation material within the cell adhesiveness variation material layer is varied by the action of the photocatalyst within the photocatalyst-comprising layer. Hence, such cell adhesiveness variation pattern can be formed, comprising portions subjected to energy irradiation and portions not subjected to energy irradiation, where such portions differ in terms of cell adhesiveness.

Members used in such cell array substrate in this embodiment will be separately explained.

### 1. Cell adhesiveness variation material layer

In the case of a cell array substrate of this embodiment, a cell adhesiveness variation material layer is formed on the substrate. Such cell adhesiveness variation material layer is similar to a layer that is formed with the use of a material explained in the above 2^{nd} embodiment. In addition, in this embodiment, a cell adhesiveness variation material layer is not principally required to comprise a photocatalyst therewithin, but may comprise a photocatalyst in a small amount in view of sensitivity and the like.

Furthermore, in this embodiment, in a manner similar to that in the above 2^{nd} embodiment, a cell adhesiveness variation material layer may be formed as a layer to be decomposed and removed (that is, the layer is subjected to decomposition and removal through the action of a photocatalyst) on a substrate. In this case, energy irradiation is carried out using a photocatalyst-comprising-layer-side base plate, so as to form regions wherein the cell adhesiveness variation material layer has been decomposed by the action of the photocatalyst along with energy irradiation (that is, the regions where the substrate has been exposed) and regions where the cell adhesiveness variation material layer has remained. Thus, a cell adhesiveness variation pattern is formed. Such type of cell adhesiveness variation material layer having the thus formed pattern is used.

### 2. Substrate

A substrate that is used in this embodiment is not particularly limited, as long as the above cell adhesiveness variation material layer can be formed. A substrate similar to that explained in the 1^{st} embodiment can be used.

### 3. Photocatalyst-comprising layer

Next, a photocatalyst-comprising layer that is used in this embodiment will be explained as follows. Such photocatalyst-comprising layer that is used in this embodiment is a layer comprising a photocatalyst. Such layer is generally formed on a base body such as glass and then used. In this embodiment, such photocatalyst-comprising layer is arranged so that the layer and the above cell adhesiveness variation material layer face each other. Through energy irradiation, the cell adhesiveness of the cell adhesiveness variation material layer can be varied by the action of the photocatalyst contained in such photocatalyst-comprising layer. In this embodiment, such photocatalyst-comprising layer is arranged at a predetermined position when energy irradiation is carried out, so that a cell adhesiveness variation pattern can be formed. Thus, there is no need to cause the above cell adhesiveness variation material layer to comprise a photocatalyst. Hence, such photocatalyst-comprising layer is advantageous in that a cell adhesiveness variation material layer can be kept free from the action of a photocatalyst over time.

Such photocatalyst-comprising layer is similar to the photocatalyst treatment layer that is explained in the above 2^{nd} embodiment.

### 4. Cell adhesiveness variation pattern

In this embodiment, a cell adhesiveness variation pattern is formed in the above cell adhesiveness variation material layer. Such cell adhesiveness variation pattern is formed by carrying out energy irradiation in a pattern using the above photocatalyst-comprising layer, so as to vary the cell adhesiveness of the surface of the cell adhesiveness variation material layer by the action of the photocatalyst in the photocatalyst-comprising layer.

### II. Method for producing a cell array substrate

Next, the method for producing a cell array substrate of the present invention will be explained. Examples of such method for producing the cell array substrate of the present invention include three embodiments as described above. All of these embodiments are characterized by the formation of a substrate for pattern formation that comprises a substrate and a layer formed on the substrate, whose adhesiveness can be varied by the action of a photocatalyst along with energy irradiation, irradiating energy to the substrate for pattern formation, so as to cause the photocatalyst to act thereon, followed by the formation of a cell adhesiveness variation pattern with variation in cell adhesiveness.

According to the method for producing the cell array substrate of the present invention, a layer whose cell adhesiveness is varied by the action of a photocatalyst along with the above energy irradiation is formed. Thus, through energy irradiation in a required pattern, it becomes possible to easily produce a cell array substrate on which a cell adhesiveness variation pattern (with variation in cell adhesiveness in a high-definition pattern) is formed. Therefore, a cell array substrate with a high-definition pattern can be produced with convenient steps without using any treatment solution that adversely affects cells. Moreover, such production method does not require any modification of a cell adhesiveness variation material. Thus, options for material selection can be expanded. Furthermore, a biological cell adhesiveness variation material that exerts specific adhesiveness described later can also be used without any problems.

The above 1^{st} to 3^{rd} embodiments for the cell array substrate of the present invention will be separately explained as follows.

### A. 1^{st} Embodiment

First, the 1^{st} embodiment of the cell array substrate of the present invention will be explained. The 1^{st} embodiment of the cell array substrate of the present invention comprises: a step of forming a substrate for pattern formation that comprises a substrate and a photocatalyst-comprising cell adhesiveness variation layer formed on the above substrate and comprising a photocatalyst and a cell adhesiveness variation material whose cell adhesiveness is varied by the action of the photocatalyst along with energy irradiation; and a step of forming a cell adhesiveness variation pattern by irradiating energy to the above photocatalyst-comprising cell adhesiveness variation layer so as to vary the cell adhesiveness of the above photocatalyst-comprising cell adhesiveness variation layer.

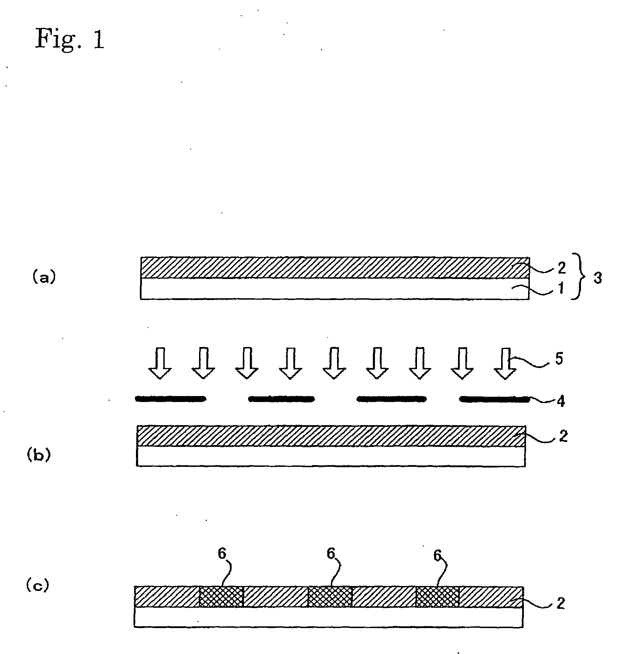

A method for producing a cell array substrate in this embodiment is carried out as shown in Fig. 1, for example. Specifically, a substrate for pattern formation 3 (the step of forming a substrate for pattern formation (Fig. 1 (a)) comprising a substrate 1 and a photocatalyst-comprising cell adhesiveness variation layer 2 formed on the substrate 1 is formed. Next, the step of forming a cell adhesiveness variation pattern (Fig. 1 (c)) is carried out by irradiating the above photocatalyst-comprising cell adhesiveness variation layer 2 with energy 5 using a photomask 4, for example (Fig. 1(b)), and then forming a cell adhesiveness variation pattern 6 wherein the cell adhesiveness of a photocatalyst-comprising cell adhesiveness variation layer 2 has been varied.

In this embodiment, a photocatalyst-comprising cell adhesiveness variation layer comprising a photocatalyst and the above cell adhesiveness variation material is formed. Through energy irradiation in the step of forming a cell adhesiveness variation pattern, the cell adhesiveness of the cell adhesiveness variation material is varied by the action of the photocatalyst within the photocatalyst-comprising cell adhesiveness variation layer. Thus a cell adhesiveness variation pattern comprising portions subjected to energy irradiation and portions not subjected to energy irradiation, where the portions differ in terms of cell adhesiveness, can be formed. Each step of this embodiment will be explained.

### 1. Step of forming a substrate for pattern formation

First, the step of forming a substrate for pattern formation in this embodiment will be explained. The step of forming a substrate for pattern formation in this embodiment is a step for forming such substrate that comprises a substrate and a photocatalyst-comprising cell adhesiveness variation layer that is formed on the substrate and comprises a photocatalyst and a cell adhesiveness variation material whose cell adhesiveness is varied by the action of the photocatalyst along with energy irradiation.

This step can be carried out by applying a coating solution comprising a photocatalyst and a cell adhesiveness variation material to a substrate by a known application method such as a spin coating, a spray coating, a dip coating, a roll coating, or a bead coating method and thus forming a photocatalyst-comprising cell adhesiveness variation layer. When a UV-hardened type component is contained as a binder, a photocatalyst-comprising layer can be formed through UV irradiation to carry out hardening treatment.

### 2. Step of forming a cell adhesiveness variation pattern

Next, the step of forming a cell adhesiveness variation pattern in this embodiment will be explained. The step of forming a cell adhesiveness variation pattern in this embodiment is a step for forming such pattern by subjecting the above photocatalyst-comprising cell adhesiveness variation layer to energy irradiation and thus forming a cell adhesiveness variation pattern wherein the cell adhesiveness of the above photocatalyst-comprising cell adhesiveness variation layer has been varied.

With this step, wherein energy irradiation is carried out in a desired pattern, the cell adhesiveness of only the regions (of a photocatalyst-comprising cell adhesiveness variation layer) subjected to energy irradiation can be varied. Furthermore, a high-definition cell adhesiveness variation pattern comprising regions having good cell adhesiveness and regions having poor cell adhesiveness can be formed.

"Energy irradiation (exposure)" used in this embodiment is a concept that includes any form of irradiation with energy rays capable of causing variation in the cell adhesiveness on the surface of a photocatalyst-comprising cell adhesiveness variation layer. Such energy irradiation is not limited to irradiation with visible light.

Light wavelength that is used for such energy irradiation is generally determined to be 400 nm or less and preferably 380 nm or less. This is because a preferable photocatalyst that is used for a photocatalyst-comprising cell adhesiveness variation layer as described above is titanium dioxide and light having a wavelength as described above is preferable as energy to activate the action of a photocatalyst with the use of such titanium dioxide.

Examples of a light source that can be used for such energy irradiation include a mercury lamp, a metal halide lamp, a xenon lamp, an excimer lamp, and other various light sources.

In addition to a method that comprises carrying out patterned irradiation via a photomask using the above light source, a method that comprises carrying out irradiation so as to draw a pattern using a laser such as excimer or YAG can also be used.

The amount of energy irradiation should be the amount of irradiation required for varying the cell adhesiveness of the surface of a photocatalyst-comprising cell adhesiveness variation layer by the action of the photocatalyst within such layer.

The cell adhesiveness of the surface of a photocatalyst-comprising cell adhesiveness variation layer is varied depending on the amount of energy irradiation. Hence, the adhesiveness can be adjusted by regulating the energy irradiation time, for example. Therefore, the surface can be prepared to have proper adhesiveness. Cell adhesiveness can be evaluated using the water contact angle on the surface as described above. Through regulation of the energy irradiation time to obtain a surface having a proper water contact angle, a surface having a proper adhesiveness can be prepared. For example, when fluoroalkyl silane is used as a cell adhesiveness variation material and the material is irradiated with ultraviolet light at 365 nm and at an intensity of 25.0 mW/second, and then quartz is used for the substrate of a photomask,a surface having preferable adhesiveness can be prepared through generally 120 to 600 seconds and preferably 240 to 480 seconds of irradiation. Such energy irradiation time, irradiation intensity, and the like can be appropriately regulated depending on a material for a substrate, a cell adhesiveness variation material, and the like to be used herein.

At this time, it is preferable to carry out energy irradiation while heating a photocatalyst-comprising cell adhesiveness variation layer. This is preferable because sensitivity can be elevated and cell adhesiveness can be varied efficiently. Specifically, heating within a range between 30°C and 80°C is preferable.

Regarding direction for energy irradiation in this embodiment, when the above substrate is transparent, patterned energy irradiation or laser irradiation to draw a pattern can also be carried out via a photomask from either the substrate side or the photocatalyst-comprising cell adhesiveness variation layer side. On the other hand, when a substrate is opaque, energy irradiation should be carried out from the photocatalyst-comprising cell adhesiveness variation layer side.

### B. 2^{nd} Embodiment

Next, the 2^{nd} embodiment of the cell array substrate of the present invention will be explained. The 2^{nd} embodiment of the cell array substrate of the present invention comprises: a step of forming a substrate for pattern formation that comprises a substrate, a photocatalyst-comprising photocatalyst treatment layer formed on the above substrate, and a cell adhesiveness variation material layer being formed on the above photocatalyst treatment layer and comprising a cell adhesiveness variation material whose cell adhesiveness is varied by the action of the photocatalyst along with energy irradiation; and a step of forming a cell adhesiveness variation pattern by subjecting the above cell adhesiveness variation material layer to energy irradiation so as to vary the cell adhesiveness of the above cell adhesiveness variation material layer.

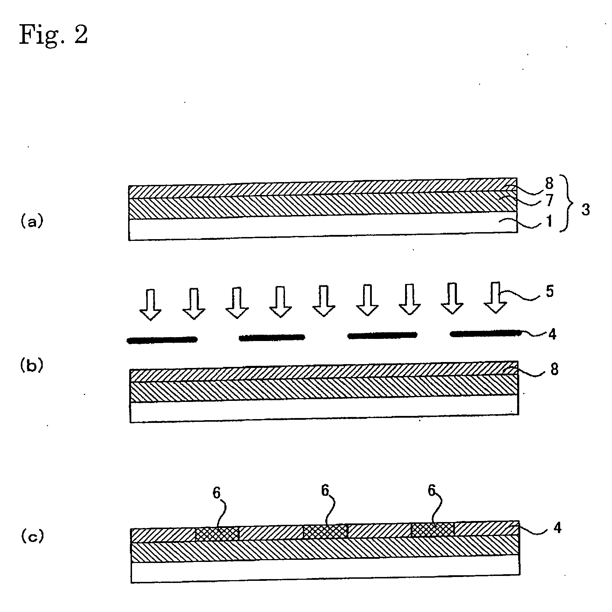

The method for producing a cell array substrate in this embodiment is carried out as shown in Fig. 2, for example. Specifically, a substrate for pattern formation 3 (the step of forming a substrate for pattern formation (Fig. 2(a)) comprising a substrate 1, a photocatalyst treatment layer 7 that is formed on the substrate 1, and a cell adhesiveness variation material layer 8 that is formed on the photocatalyst treatment layer 7 is formed. Next, the step of forming a cell adhesiveness variation pattern (Fig. 2(c)) is carried out by irradiating the above cell adhesiveness variation material layer 8 with energy 5 using a photomask 4, for example (Fig. 2(b)), and then forming a cell adhesiveness variation pattern 6 wherein the cell adhesiveness of the cell adhesiveness variation material layer 8 has been varied.

In this embodiment, a photocatalyst treatment layer and the above cell adhesiveness variation material layer are formed. Through energy irradiation in the step of forming a cell adhesiveness variation pattern, the cell adhesiveness within the cell adhesiveness variation material layer is varied by the action of the photocatalyst contained in the photocatalyst treatment layer. Thus, a cell adhesiveness variation pattern comprising portions subjected to energy irradiation and portions not subjected to energy irradiation, where the portions differ in terms of cell adhesiveness, can be formed. Each step of this embodiment will be explained as follows.

### 1. Step of forming a substrate for pattern formation

First, the step of forming a substrate for pattern formation in this embodiment will be explained. The step of forming a substrate for pattern formation in this embodiment is a step for forming such substrate that comprises a photocatalyst-comprising photocatalyst treatment layer formed on the above substrate and a cell adhesiveness variation material layer that is formed on the above photocatalyst treatment layer and comprises a cell adhesiveness variation material whose cell adhesiveness is varied by the action of the photoctalyst along with energy irradiation.

Such photocatalyst treatment layer that is formed in this step may consist of a photocatalyst alone or may be formed by mixture with a binder.

Examples of a method for forming a photocatalyst treatment layer consisting of a photocatalyst alone include a sputtering method, a CVD method, and a vacuum film production method such as a vacuum deposition method. For example, a method that is used when a photocatalyst is titanium dioxide comprises forming amorphous titania on a substrate and then causing a phase change through calcination to obtain crystalline titania. Formation of a photocatalyst treatment layer by the vacuum film production method enables preparation of a photocatalyst treatment layer formed of a uniform film and comprising a photocatalyst alone. Thus, the cell adhesiveness on a cell adhesiveness variation material layer can be uniformly varied. Furthermore, such layer consists of a photocatalyst alone. Thus, it becomes possible to vary the cell adhesiveness on a cell adhesiveness variation material layer more efficiently than in the case of using a binder.

Furthermore, when a photocatalyst treatment layer is prepared by mixing a photocatalyst with a binder, such layer can be formed by preparing an application solution by dispersing such photocatalyst and such binder in a solvent together with another additive if necessary and then applying the thus prepared application solution to a transparent substrate. A solvent that is used herein is preferably an alcohol-based organic solvent such as ethanol or isopropanol. Application can be carried out by a known application method such as a spin coating, a spray coating, a dip coating, a roll coating, or a bead coating method. When a UV-hardened type component is contained as a binder, a photocatalyst treatment layer can be formed through UV irradiation to carry out hardening treatment.

Subsequently, a coating solution comprising the above cell adhesiveness variation material is applied onto the above photocatalyst treatment layer by a known application method such as a spin coating, a spray coating, a dip coating, a roll coating, or a bead coating method. Thus, a cell adhesiveness variation material layer can be formed. When a UV-hardened type component is contained as a binder, a photocatalyst treatment layer can be formed through UV irradiation to carry out hardening treatment.

Such substrate, such photocatalyst treatment layer, and such cell adhesiveness variation material layer that are used in this step are similar to those explained in the above section of the 2^{nd} embodiment, "I. Cell array substrate."

### 2. Step of forming a cell adhesiveness variation pattern

Next, the step of forming a cell adhesiveness variation pattern in this embodiment will be explained. The step of forming a cell adhesiveness variation pattern in this embodiment is a step for forming such pattern by subjecting the above cell adhesiveness variation material layer to energy irradiation and thus forming a cell adhesiveness variation pattern wherein the cell adhesiveness of the above cell adhesiveness variation material layer has been varied.

With this step wherein energy irradiation is carried out in a desired pattern, the cell adhesiveness of regions (of a cell adhesiveness variation material layer) subjected to energy irradiation can be exclusively varied. Furthermore, a high-definition cell adhesiveness variation pattern can be formed, which comprises regions having good cell adhesiveness and regions having poor cell adhesiveness.

An energy irradiation method, energy to be used for irradiation, and the amount of energy irradiation that are used in this step are similar to those in the above 1^{st} embodiment.

### C. 3^{rd} Embodiment

Next, the 3^{rd} embodiment of the cell array substrate of the present invention will be explained. The 3^{rd} embodiment of the cell array substrate of the present invention comprises: a step of forming a substrate for pattern formation that comprises a substrate and a cell adhesiveness variation material layer that is formed on the substrate and comprises a cell adhesiveness variation material whose cell adhesiveness is varied by the action of a photocatalyst along with energy irradiation; and a step of forming a cell adhesiveness variation pattern by arranging the above substrate for pattern formation and a photocatalyst-comprising-layer-side base plate that comprises a photocatalyst-comprising layer and a base body so that the above cell adhesiveness variation material layer and the above photocatalyst-comprising layer face each other, carrying out energy irradiation from a predetermined direction, and thus forming a cell adhesiveness variation pattern wherein the cell adhesiveness of the above cell adhesiveness variation material layer has been varied.

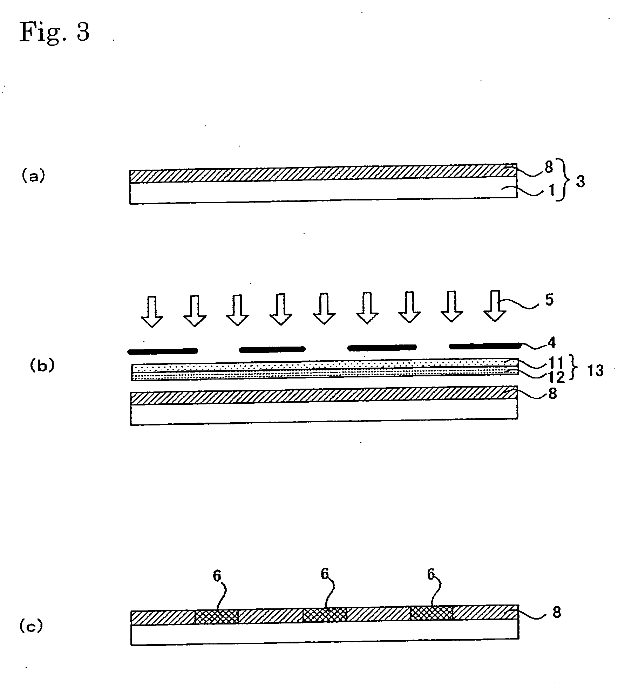

The method for producing a cell array substrate in this embodiment is carried out as shown in Fig. 3, for example. Specifically, a substrate for pattern formation 3 is formed (the step of forming a substrate for pattern formation (Fig. 3(a)), which comprises a substrate 1 and a cell adhesiveness variation material layer 8 formed on the substrate 1. Next, a photocatalyst-comprising-layer-side base plate 13 is prepared, which comprises a base body 11 and a photocatalyst-comprising layer 12 formed on the base body 11. The step of forming a cell adhesiveness variation pattern is carried out (Fig. 3(c)) by arranging the photocatalyst-comprising layer 12 in the photocatalyst-comprising-layer-side base plate 13 and the above cell adhesiveness variation material layer 8 so that the layers face each other, irradiating with energy 5 using a photomask 4, for example (Fig. 3(b)), and then forming a cell adhesiveness variation pattern 6 wherein the cell adhesiveness of the cell adhesiveness variation material layer 8 has been varied.

In this embodiment, the above cell adhesiveness variation material layer is formed. Through energy irradiation using the photocatalyst-comprising layer-side base plate in the step of forming a cell adhesiveness variation pattern, the cell adhesiveness within the cell adhesiveness variation material layer is varied by the action of the photocatalyst contained in the photocatalyst-comprising layer. Thus a cell adhesiveness variation pattern can be formed, which comprises portions subjected to energy irradiation and portions not subjected to energy irradiation, where the portions differ in terms of cell adhesiveness. Each step of this embodiment will be explained.

### 1. Step of forming a substrate for pattern formation

First, the step of forming a substrate for pattern formation in the present invention will be explained. The step of forming a substrate for pattern formation in the present invention is a step for forming such substrate that comprises a substrate and a cell adhesiveness variation material layer that is formed on the substrate and comprises a cell adhesiveness variation material whose cell adhesiveness is varied by the action of a photocatalyst along with energy irradiation.

This step can be carried out by applying a coating solution comprising a cell adhesiveness variation material to a substrate by a known application method such as a spin coating, a spray coating, a dip coating, a roll coating, or a bead coating method and thus forming a cell adhesiveness variation material layer. When a UV-hardened type component is contained as a binder, a photocatalyst-comprising layer can be formed through UV irradiation to carry out hardening treatment.

Such substrate and such cell adhesiveness variation material that can be used in this step are similar to those explained in the above section of the 1^{st} embodiment, "I. Cell array substrate."

### 2. Step of forming a cell adhesiveness variation pattern

Next, the step of forming a cell adhesiveness variation pattern in this embodiment will be explained. The step of forming an adhesiveness variation pattern in this embodiment is a step for forming such pattern by arranging the above substrate for pattern formation and a photocatalyst-comprising-layer-side base plate that comprises a photocatalyst-comprising layer and a base body so that the above cell adhesiveness variation material layer and the above photocatalyst-comprising layer face each other, and then carrying out energy irradiation from a predetermined direction, so as to form a pattern wherein the cell adhesiveness of a cell adhesiveness variation material layer has been varied.

With this step wherein a photocatalyst-comprising layer in a photocatalyst-comprising-layer-side base plate and a cell adhesiveness variation material layer are arranged so that the layers face each other and energy irradiation is carried out in a desired pattern, the cell adhesiveness of regions (of a cell adhesiveness variation material layer) subjected to energy irradiation can be exclusively varied. Furthermore, a high-definition cell adhesiveness variation pattern can be formed, which comprises regions having good cell adhesiveness and regions having poor cell adhesiveness.

Such photocatalyst-comprising-layer-side base plate and energy irradiation that are used and carried out, respectively, in this step will be separately explained as follows.

### (1) Photocatalyst-comprising-layer-side base plate

First, a photocatalyst-comprising-layer-side base plate that is used in this embodiment will be explained.

Such photocatalyst-comprising-layer-side base plate that is used in this embodiment comprises at least a photocatalyst-comprising layer and a base body. Such base plate is generally prepared by forming a photocatalyst-comprising layer in the shape of thin film (formed by a predetermined method) on a base body. Furthermore, a photocatalyst-comprising-layer-side base plate that can also be used herein may comprise patterned photocatalyst-comprising-layer-side shielding portions or a primer layer formed thereon.

In this embodiment, at the time of energy irradiation, the above cell adhesiveness variation material layer and the photocatalyst-comprising layer in the above photocatalyst-comprising-layer-side base plate are arranged so that the layers face each other with a predetermined space between the two. The cell adhesiveness of the cell adhesiveness variation material layer is varied by the action of the photocatalyst-comprising layer of the photocatalyst-comprising-layer-side base plate. The photocatalyst-comprising-layer-side base plate is removed after energy irradiation, so that a cell adhesiveness variation pattern is formed. Each component of such photocatalyst-comprising-layer-side base plate will be explained.

### a. Photocatalyst-comprising layer

A photocatalyst-comprising layer that is used in this embodiment comprises at least a photocatalyst and may or may not comprise a binder. The photocatalyst-comprising layer is similar to the photocatalyst treatment layer described in the above 2^{nd} embodiment.

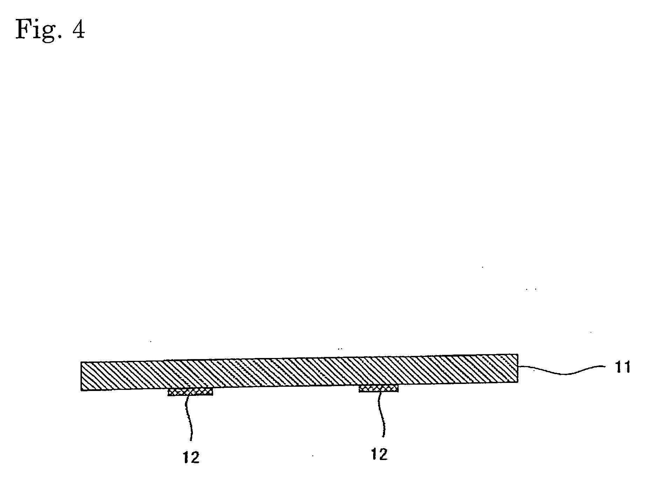

Such photocatalyst-comprising layer that is used in this embodiment may be, as shown in Fig. 3, for example, a layer formed on the whole surface of a base body 11. For example, as shown in Fig. 4, the photocatalyst-comprising layer may be a photocatalyst-comprising layer 12 patterned on the base body 11.

By patterning of such photocatalyst-comprising layer, patterned irradiation using a photomask or the like is not required at the time of energy irradiation. Furthermore, through irradiation of the whole surface, a cell adhesiveness variation pattern can be formed in a cell adhesiveness variation material layer.

A patterning method for such photocatalyst-comprising layer is not particularly limited. For example, such patterning can be carried out by a photolithography method or the like.

Furthermore, energy irradiation is carried out while closely contacting a photocatalyst-comprising layer and a cell adhesiveness variation material layer with each other. In such case, the properties of only portions where the photocatalyst-comprising layer has been actually formed are varied. Thus, such case is advantageous in that energy irradiation may be carried out from any direction, as long as portions where the above photocatalyst-comprising layer and cell adhesiveness variation material layer face each other are irradiated with energy. Another advantage is that energy to be used for irradiation is also not particularly limited to parallel energy such as parallel light.

### b. Base body

In this embodiment, as shown in Fig. 3, the photocatalyst-comprising-layer-side base plate 13 comprises at least a base body 11 and the photocatalyst-comprising layer 12 formed on the base body 11. At this time, the material composing a base body used herein is appropriately selected depending on the direction of energy irradiation described later, necessity for the transparency of the thus obtained cell array substrate, and the like.

Furthermore, such base body that is used in this embodiment may be a base body having flexibility, such as a film made of a resin, or a base body lacking flexibility, such as a glass substrate. Moreover, as another type of base body, an optical waveguide such as optical fiber can also be used. Such base body can be appropriately selected depending on the energy irradiation method.

In addition, to improve close contact between the surface of a base body and a photocatalyst-comprising layer, an anchor layer may also be formed on the base body. Examples of such anchor layer include a silane coupling agent and a titanium coupling agent.

### c. Photocatalyst-comprising-layer-side shielding portion

A photocatalyst-comprising-layer-side base plate that is used in this embodiment may comprise photocatalyst-comprising-layer-side shielding portions patterned thereon. With the use of such photocatalyst-comprising-layer-side base plate comprising the photocatalyst-comprising-layer-side shielding portions, it is not required to use a photomask or to carry out laser irradiation to draw a pattern at the time of energy irradiation. Furthermore, it is not required to carry out positioning for a photocatalyst-comprising-layer-side base plate and a photomask. Hence, a convenient step can be realized and no expensive apparatuses are needed for drawing irradiation. Thus, the use of such photocatalyst-comprising-layer-side base plate is advantageous in terms of cost.

The following two embodiments are possible for such photocatalyst-comprising-layer-side base plate comprising such photocatalyst-comprising-layer-side shielding portions, depending on the positions at which the photocatalyst-comprising-layer-side shielding portions are formed.

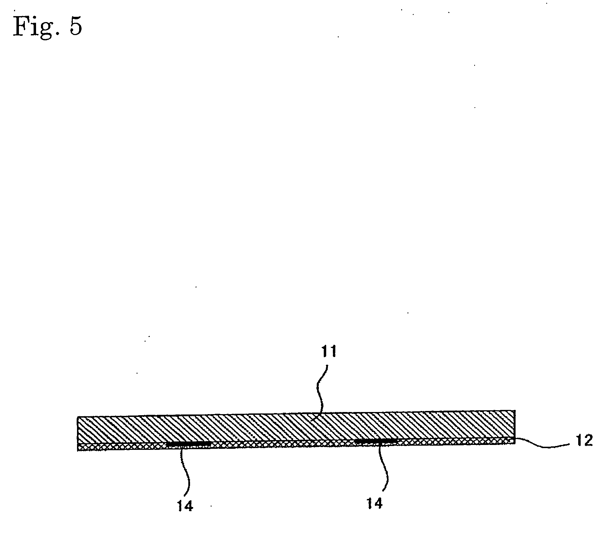

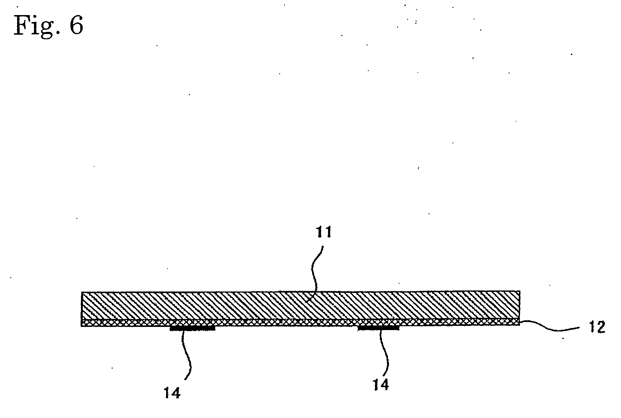

One embodiment of the photocatalyst-comprising-layer-side base plate is prepared as shown in Fig. 5, for example, wherein photocatalyst-comprising-layer-side shielding portions 14 are formed on the base body 11 and the photocatalyst-comprising layer 12 is formed on the photocatalyst-comprising-layer-side shielding portions 14. The other embodiment of the photocatalyst-comprising-layer-side base plate is prepared as shown in Fig. 6, for example, wherein the photocatalyst-comprising layer 12 is formed on the base body 11 and the photocatalyst-comprising-layer-side shielding portions 14 are formed on the photocatalyst-comprising layer 12.

In both embodiments, compared with the case of using a photomask, photocatalyst-comprising-layer-side shielding portions are arranged in the vicinity of portions where the above photocatalyst-comprising layer and cell adhesiveness variation material layer are to be arranged. Thus, the effect of energy scattering within a base body and the like can be reduced. Thus, it becomes possible to carry out patterned energy irradiation in an extremely precise manner.

Furthermore, in the above embodiment where photocatalyst-comprising-layer-side shielding portions are formed on a photocatalyst-comprising layer, when a photocatalyst-comprising layer and a cell adhesiveness variation material layer are arranged at predetermined positions, the film thickness of each photocatalyst-comprising-layer-side shielding portion is prepared to be the same as the width of the space between the two layers. Hence, the embodiment is advantageous in that the above photocatalyst-comprising-layer-side shielding portions can also be used as a spacer to maintain the above space at a constant width. Moreover, when the height of such portion as a spacer is insufficient, another spacer may be separately provided at the shielding portions.

Specifically, when the above photocatalyst-comprising layer and cell adhesiveness variation material layer are arranged so that the layers face each other with a predetermined space, the above photocatalyst-comprising-layer-side shielding portions and cell adhesiveness variation material layer can be arranged in close contact. This makes it possible to precisely obtain the above predetermined space. Furthermore, through energy irradiation from the photocatalyst-comprising-layer-side base plate under such state, it becomes possible to precisely form a cell adhesiveness variation pattern on the cell adhesiveness variation material layer.

A method for forming such photocatalyst-comprising-layer-side shielding portions is not particularly limited. Such method is appropriately selected and used depending on the properties of the surface on which photocatalyst-comprising-layer-side shielding portions are formed, shielding property as required against energy, and the like.

For example, such photocatalyst-comprising-layer-side shielding portions may be formed by forming a metal thin film made of chrome or the like with a thickness between approximately 1000 Å and 2000 Å by a sputtering method, a vacuum deposition method, or the like and then patterning the thin film. A general patterning method such as a sputtering method can be used as such patterning method.

Furthermore, such patterning method may also be a method that comprises preparing a layer that comprises shielding particles such as carbon fine particles, metallic oxide, an inorganic pigment, or an organic pigment in a resin binder and then patterning such layer. Examples of such resin binder that is used herein include 1 type of or a mixture of 2 or more types of resins such as a polyimide resin, an acrylic resin, an epoxy resin, polyacryl amide, polyvinyl alcohol, gelatin, casein, and cellulose, and a photosensitive resin. Furthermore, an O/W emulsion type resin composition such as an emulsified reactive silicone can be used. The thickness of each shielding portion made of resin can be determined within a range between 0.5 µm and 10 µm. As a patterning method used for such shielding portions made of resin, a generally employed method such as a photolithography method, printing method, or the like can be used.

Two possible positions of photocatalyst-comprising-layer-side shielding portions are explained in the above explanation. One of such position is between a base body and a photocatalyst-comprising layer. The other one is the surface of a photocatalyst-comprising layer. In addition to such positions, an embodiment that can also be employed comprises forming photocatalyst-comprising-layer-side shielding portions on the surface of a base body on the side where no photocatalyst-comprising layer is formed. In this embodiment, a photomask may be contacted closely but removably to such surface, for example. Such embodiment can be appropriately used for a case where a cell adhesiveness variation patterns is changed between small lots.

### d. Primer layer

Next, a primer layer that is used for a photocatalyst-comprising-layer-side base plate in this embodiment will be explained. In this embodiment, when photocatalyst-comprising-layer-side shielding portions are patterned on a base body as described above and then a photocatalyst-comprising layer is formed thereon, so as to prepare a photocatalyst-comprising-layer-side base plate, a primer layer may be formed between the above photocatalyst-comprising-layer-side shielding portions and photocatalyst-comprising layer.

The action and functions of such primer layer are not always clear. Through the formation of the primer layer between photocatalyst-comprising-layer-side shielding portions and a photocatalyst-comprising layer, it is thought that the primer layer exhibits a function to prevent the diffusion of impurities (that are factors that inhibit variation in cell adhesiveness of a cell adhesiveness variation material layer caused by the action of a photocatalyst) coming from inside of the photocatalyst-comprising-layer-side shielding portions or each opening existing between the photocatalyst-comprising-layer-side shielding portions. Particular examples of such impurities include residues and impurities such as metal and metal ions that are generated at the time of patterning of the photocatalyst-comprising-layer-side shielding portions. Therefore, by the formation of such primer layer, treatment for causing variation in cell adhesiveness can proceed with high sensitivity. As a result, it becomes possible to obtain a pattern with high resolution.

In addition, such primer layer in this embodiment prevents impurities (existing not only on the photocatalyst-comprising-layer-side shielding portions but also at an opening formed between such photocatalyst-comprising-layer-side shielding portions) from affecting the action of a photocatalyst. The primer layer is preferably formed on the whole surface of the photocatalyst-comprising-layer-side shielding portions including the opening.

Such primer layer in this embodiment is not particularly limited, as long as it is formed such that photocatalyst-comprising-layer-side shielding portions of a photocatalyst-comprising-layer-side base plate and a photocatalyst-comprising layer do not contact with each other.

A material composing such primer layer is not particularly limited. An inorganic material hardly decomposed by the action of a photocatalyst is preferred. A specific example of such material is amorphous silica. When such amorphous silica is used, a precursor of such amorphous silica is a silicon compound represented by general formula SiX₄, where X indicates halogen, a methoxy group, an ethoxy group, an acetyl group, or the like. Hydrolysates of such compound, such as silanol or polysiloxane with an average molecular weight of 3000 or less, are preferable.

Furthermore, the film thickness of such primer layer is preferably within the range between 0.001 µm and 1 µm and particularly preferably within the range between 0.001 µm and 0.1 µm.

### (2) Energy irradiation

Next, energy irradiation in this step will be explained. In this embodiment, the above cell adhesiveness variation material layer and the above photocatalyst-comprising layer of the above photocatalyst-comprising-layer-side base plate are arranged so that the layers face each other, and then energy irradiation is carried out from a predetermined direction. Thus, a pattern with variation in cell adhesiveness of the cell adhesiveness variation material layer can be formed.

The above expression "are arranged" means a state where the layers are arranged so that a photocatalyst substantially acts on the surface of the cell adhesiveness variation material layer. The term also means, in addition to a state where the layers are caused to come into actual and physical contact, a state where the above photocatalyst-comprising layer and the above cell adhesiveness variation material layer are arranged with a predetermined space. Such space is preferably 200 µm or less.

The above space in this embodiment is particularly within the range between 0.2 µm and 10 µm and preferably within the range between 1 µm and 5 µm in consideration of extremely good patterning accuracy, high photocatalyst sensitivity, and good efficiency of causing variation in the cell adhesiveness of a cell adhesiveness variation material layer. The space within such range is particularly effective for a cell adhesiveness variation material layer with a small area that enables control of such space with particularly high accuracy.

On the other hand, when a cell adhesiveness variation material layer with an area that is as large as 300 mm x 300 mm or greater, for example, is treated, it is extremely difficult to form a fine space as described above between a photocatalyst-comprising-layer-side base plate and a cell adhesiveness variation material layer without causing them to come into contact. Therefore, when a cell adhesiveness variation material layer has relatively a large area, the above space is preferably within a range between 10 µm and 100 µm and particularly preferably within the range between 10 µm and 20 µm. The space set to be within such range can have effects of: causing no problems such as lowered patterning accuracy (e.g., a blur patterning), deteriorated photocatalyst sensitivity, and lower efficiency of causing variation in cell adhesiveness as a result of such deteriorated sensitivity; and not generating uneven variation in the cell adhesiveness on a cell adhesiveness variation material layer.

When such cell adhesiveness variation material layer with a relatively large area is subjected to energy irradiation, within an apparatus for energy irradiation, the space between a photocatalyst-comprising-layer-side base plate and a cell adhesiveness variation material layer is preferably set in an apparatus for positioning the plate and the layer within the range between 10 µm and 200 µm and particularly preferably within the range between 10 µm and 20 µm. Determination of the space within such a range makes it possible to arrange a photocatalyst-comprising-layer-side base plate and a cell adhesiveness variation material layer without causing any drastic decrease in patterning accuracy or in photocatalyst sensitivity, and without the two coming into contact.

A photocatalyst-comprising layer and the surface of a cell adhesiveness variation material layer are arranged with a predetermined space as described above. Thus, removal of active oxygen species generated by the action of oxygen, water, and a photocatalyst is facilitated. Specifically, when the space between a photocatalyst-comprising layer and a cell adhesiveness variation material layer is narrower than those within the above ranges, it becomes difficult to remove the above active oxygen species. As a result, the rate of causing variation in cell adhesiveness may be lowered. Thus, such narrow space is not preferable. Furthermore, arrangement with a space wider than those within the above ranges makes it difficult for the generated active oxygen species to reach the cell adhesiveness variation material layer. This case is also not preferable because this may result in a lower rate of causing variation in cell adhesiveness.

An example of a method for arranging a photocatalyst-comprising layer and a cell adhesiveness variation material layer with uniform and extremely narrow space is a method that uses a spacer. A uniform space can be formed with the use of a spacer. Furthermore, portions (of the surface of a cell adhesiveness variation material layer) to which the spacer is caused to come into contact are free from the action of a photocatalyst. Hence, the spacer is prepared to have a pattern similar to that of the above cell adhesiveness variation pattern, so that a predetermined cell adhesiveness variation pattern can be formed on a cell adhesiveness variation material layer.

In this embodiment, such arrangement should be maintained at least during energy irradiation.

Types of energy to be used for irradiation, irradiation method, the amount of energy irradiation, and the like are similar to those explained in the above 1^{st} embodiment.

In addition, the present invention is not limited to the above embodiments. The above embodiments are provided for illustrative purposes. Any embodiment that has substantially the same constitution as that of the technical idea disclosed in the claims of the present invention and exerts action and effects similar to those exerted by the present invention is encompassed within the technical scope of the present invention.

### III. Cell adhesion substrate

The method for culturing cells of the present invention comprises the steps of: causing a cell adhesion substrate comprising a cell array substrate having cells adhered thereto in a pattern to closely contact to a cell culture substrate; and transferring the cells adhering to the cell array substrate onto the cell culture substrate while the cells are in such patterned state. The step of transferring cells from a cell adhesion substrate and the step of culturing the cells will be explained as follows.

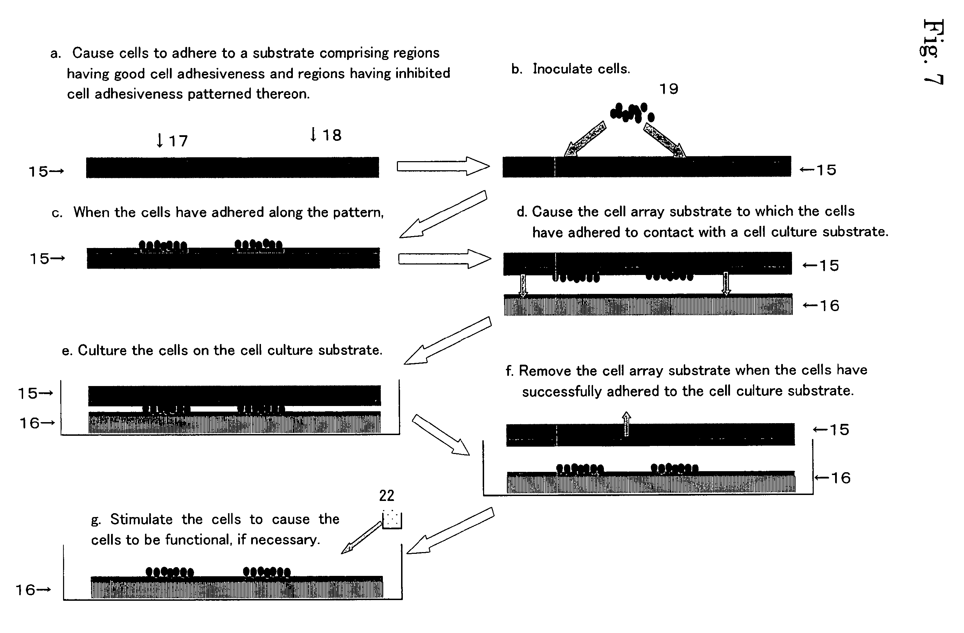

As an example, an outline of an embodiment is shown in Fig. 7. Cells are inoculated on a cell array substrate (15) comprising regions having good cell adhesiveness (17) and regions having inhibited cell adhesiveness (18) patterned thereon. The cells are caused to adhere to form a pattern, so that a cell adhesion substrate is prepared. Subsequently, the cell adhesion substrate is caused to closely contact to a cell culture substrate (16), so as to transfer the cells. The cells are then cultured. If necessary, the cells are stimulated with a cell stimulating factor (22).

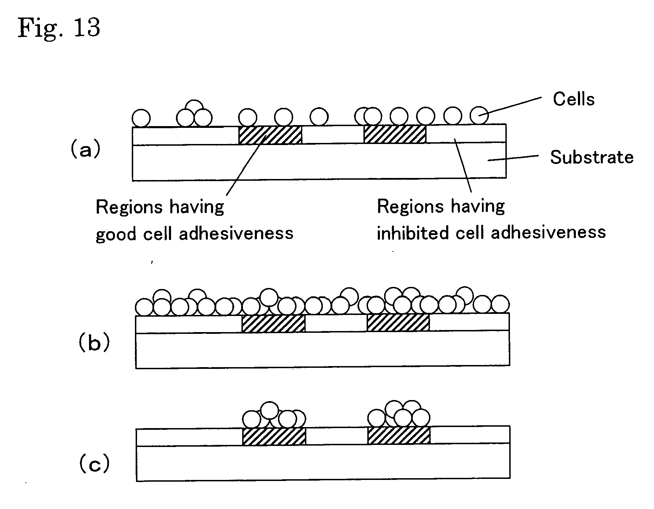

Next, the cell adhesion substrate of the present invention will be explained. As shown in Fig. 13 (c), the cell adhesion substrate of the present invention is the above cell array substrate having a cell adhesiveness variation pattern (that comprises regions having good cell adhesiveness and regions having inhibited cell adhesiveness, where the regions differ in terms of cell adhesiveness) formed on the substrate, where cells adhere to the regions having good cell adhesiveness.

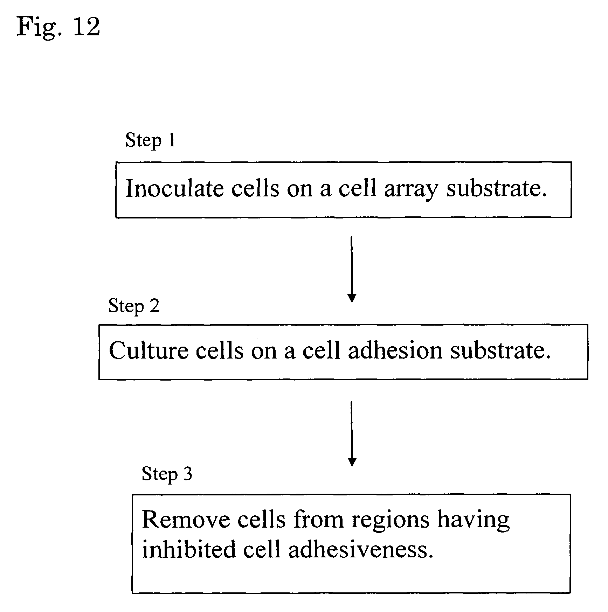

Procedures of a method for preparing the cell adhesion substrate according to the present invention are shown in Fig. 12. The states of the cell adhesion substrate at each step of the procedures are shown in Fig. 13. The cell array substrate of the present invention has a cell adhesiveness variation pattern comprising regions having good cell adhesiveness and regions having inhibited cell adhesiveness as described above. As shown in Fig. 13 (a), cells are uniformly inoculated on the surface of the cell array substrate (step 1). As shown in Fig. 13 (b), the cells are cultured for a certain time period (step 2) and then the substrate is washed to remove excessive cells existing on the regions having inhibited cell adhesiveness (step 3). As shown in Fig. 13 (c), a cell adhesion substrate having a cell pattern wherein cells adhere to the regions having good cell adhesiveness but no cells adhere to the regions having inhibited cell adhesiveness can be obtained.

A pattern on the cell adhesion substrate of the present invention is not particularly limited and is determined depending on the purpose for use or the subject for which each pattern is used. For example, regarding a capillary plexus or a nerve net, an artificially designed pattern may be used or a pattern designed based on a pattern actually existing *in vivo* may also be used. For example, a pattern designed arbitrarily depending on the size or the shape of a subject that is subjected to transplantation may be used. A preferable example of a pattern in view of good usability for a practitioner is a simple pattern that comprises regions having inhibited cell adhesiveness that are arranged completely or partially surrounding regions having good cell adhesiveness. The presence of an appropriate area of regions having no cells adhering thereto is advantageous in treatment or transfer for the next step, because such regions can be picked up using medical equipment or the like.

Cells that are inoculated on the cell array substrate are not particularly limited. The present invention is appropriately used for cells which has adhesiveness. Examples of such cells include hepatocytes that are hepatic parenchymal cells, endothelial cells such as vascular endothelial cells and corneal endothelial cells, fibroblasts, epidermal cells such as epidermal keratinocytes, epithelial cells such as bronchial epithelial cells, gastrointestinal epithelial cells, and cervical epithelial cells, mammary glandular cells, muscle cells such as smooth muscle cells and cardiac muscle cells, renal cells, pancreatic islet cells, nerve cells such as peripheral nerve cells and optic nerve cells, cartilage cells, and bone cells. The present invention is particularly preferably used for vascular endothelial cells. Vascular endothelial cells may be cells isolated and cultured from existing vessels or may be vascular endothelial cells obtained by culture to cause differentiation. Specific examples of existing vessels include vessels ranging from large vessels to micro vessels such as carotid arteries, umbilical veins, and vessels in reticular tissues. Examples of cells that are cultured to differentiate into endothelial cells include progenitor cells of vascular endothelial cells existing in the bone marrow, cord blood, and peripheral blood, adipose cells, and ES cells.

Appropriate cells can be selected according to the purpose of cell functionalization by transferring and culturing cells on the cell culture substrate. These cells may be primary cells that have been directly collected from a tissue or an organ or may be cells obtained by successive culture through several generations. Furthermore, cells that are cultured in the present invention may be any of ES cells that are undifferentiated cells, multipotent stem cells having multipotency, unipotent stem cells having unipotency, and cells that have completed differentiation.

A culture sample containing a target cell is preferably previously subjected to diffusion treatment by which a biological tissue is finely fragmented and diffused in a liquid or separation treatment by which cells other than the target cell and impurities such as cell debris in a biological tissue are removed.

Prior to inoculation of cells on the cell array substrate, it is preferable to increase the number of the target cells through preliminary culture of the cells contained in a culture sample by any one of a variety of culture methods. Examples of a general method that can be employed for such preliminary culture include a monolayer culture method, a coated dish culture method, and a gel culture method. Regarding preliminary culture, one culture method that comprises causing cells to adhere to the surface of a support and then culturing the cells is a means that is already known as namely the monolayer culture method. Specifically, for example, when a culture sample and a culture solution are placed in a culture container and then maintained under certain environmental conditions, specific viable cells alone will grow while adhering to the surface of a support such as the culture container. An apparatus, treatment conditions, and the like to be used herein are employed according to the general monolayer culture method and the like. As a material employed for the surface of a support on which cells adhere and grow, a material (e.g., polylysine, polyethyleneimine, collagen, and gelatin) with which cell adhesion and cell growth can be successfully carried out is selected. Furthermore, a chemical substance (namely, a cell adhesion factor) with which cell adhesion and cell growth can be successfully carried out is previously applied to the surface of a support such as a glass plate, a plastic plate, a slide glass, a cover glass, a plastic sheet, or a plastic film.

After culture, the culture solution within the culture container is removed, thereby removing unnecessary components such as massive and fibrous impurities that do not adhere to the surface of the support in the culture sample. Hence, only the viable cells adhering to the surface of the support can be harvested. Means such as EGTA-trypsinization can be applied for harvesting viable cells adhering to the surface of the support.

As shown in Fig. 13(a), the above preliminarily cultured cells are inoculated on the cell array substrate in a culture solution. A method for cell inoculation and an inoculation amount are not particularly limited. For example, a method disclosed in "Tissue Culture Technology (Soshiki Baiyo no Gijutu)" (edited by The Japanese Tissue Culture Association, pp. 266 to 270, issued by Asakura Pub. Co., 1999) can be used. It is preferable to inoculate cells in a sufficient amount so that the cells are not required to grow on the cell array substrate and so that the cells adhere to the substrate in the form of monolayer. It is generally preferable to inoculate cells on the order of 10⁴ to 10⁶ cells per ml of a culture solution (so that 1 ml of a culture solution contains 10⁴ to 10⁶ cells). Furthermore, it is preferable to inoculate cells on the order of 10⁴ to 10⁶ cells per 1 cm² of the cell array substrate (so that 10⁴ to 10⁶ cells are contained per cm² of the cell array substrate). This is because tissue formation by cells is inhibited when cells aggregate, and even when cells are transferred to and then cultured on a cell culture substrate, their functions will be lowered. Specifically, approximately 2 x 10⁵ cells are inoculated per 400 mm².

It is preferable to cause cells to adhere to regions having good cell adhesiveness through culture of the cells that have been inoculated on a cell array substrate in a culture solution. As a culture solution, a medium that is generally used in the technical field can be used. According to cell types to be used herein, a basic medium disclosed in "Tissue Culture Technology (Soshiki Baiyo no Gijutu)" (edited by The Japanese Tissue Culture Association, issued by Asakura Pub. Co., 3rd ed., p. 581) can be used. Examples of such medium include a MEM medium, a BME medium, a DME medium, an αMEM medium, an IMEM medium, an ES medium, a DM-160 medium, Fisher medium, an F12 medium, a WE medium, and an RPMI medium. Furthermore, these media supplemented with a serum component (e.g., fetal calf serum) or the like and commercial serum free media such as a Gibco serum free medium (Invitrogen Corp.) can be used.

As shown in Fig. 13(b), a purpose of the step of culturing cells is to cause cells to adhere to regions having good cell adhesiveness of a cell array substrate. Time for culturing cells is generally between 18 hours and 30 hours and preferably between 20 hours and 24 hours. When cells are cultured for a proper time period, cells on regions having inhibited cell adhesiveness of the cell array substrate are washed away (upon washing of the substrate). On the other hand, cells on regions having good cell adhesiveness will remain on the cell array substrate because of appropriate adhesiveness. Thus, it becomes possible to easily transfer the remaining cells to a cell culture substrate.

The temperature for culture is generally 37°C. Cells are preferably cultured under a CO₂ atmosphere using a CO₂ cell culture incubator. After culture, the cell array substrate is washed, so as to wash off cells that have not adhered to the substrate. Thus, the cell adhesion substrate of the present invention can be prepared, wherein cells have been arrayed in a pattern.

When a cell array substrate has a cell adhesiveness variation pattern that comprises regions each having optimal cell adhesiveness to each cell type to be arrayed in a pattern, a plurality of types of cells can be caused to adhere to and patterned as desired on the same cell array substrate.

### IV. Transfer and culture of cells

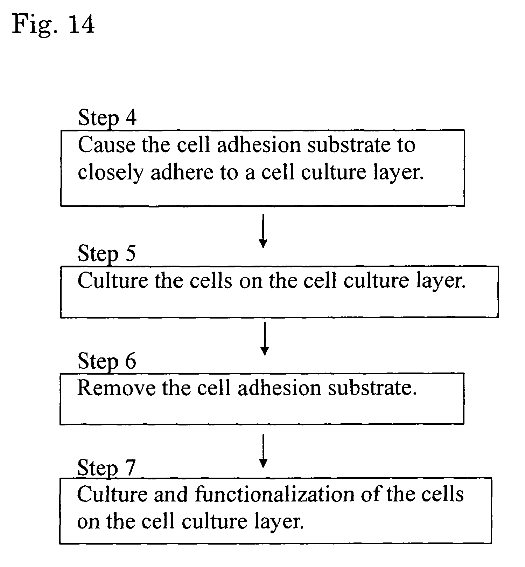

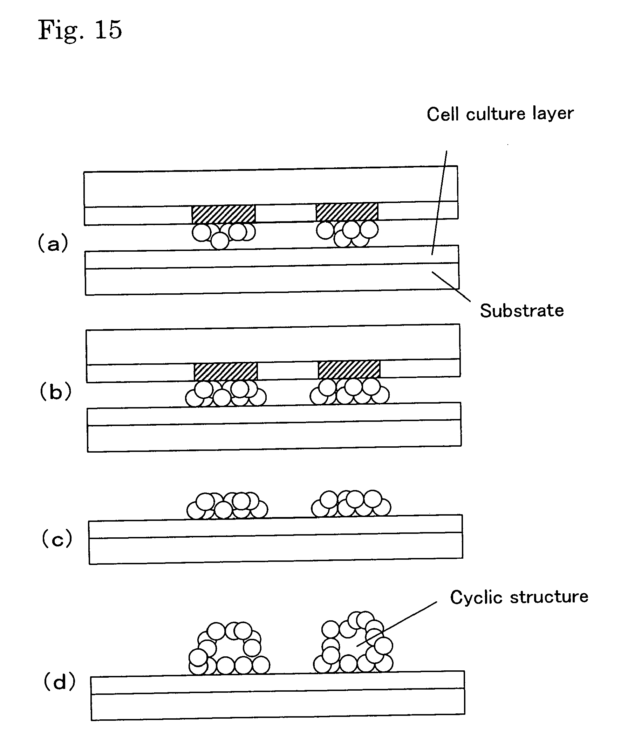

Procedures of a method for transferring and culturing cells according to the present invention are shown in Fig. 14. The states of the cell adhesion substrate and of the cell culture substrate at each step of these procedures are shown in Fig. 15.

As shown in Fig. 15(a), a cell adhesion substrate wherein cells have adhered to regions having good cell adhesiveness of a cell array substrate is caused to closely contact to a cell culture layer of a cell culture substrate (step 4). Subsequently, as shown in Fig. 15(b), the cells are cultured so that the cells adhere to the cell culture layer of the cell culture substrate (step 5). Furthermore, adhesiveness of the cells to the cell culture layer is greater than that to the regions having good cell adhesiveness. Thus, as shown in Fig. 15(c), when the cell array substrate is removed from the cell culture substrate, the cells are transferred to the cell culture substrate (step 6). When the thus transferred cells are further cultured, the cells are caused to become functional as shown in Fig. 15(d). If the cells are vascular endothelial cells, a cyclic structure is regenerated (step 7).

A cell culture substrate to which cells are transferred is not particularly limited, as long as it enables cells to adhere thereto and enables cells to be cultured. A preferable cell culture substrate comprises a cell culture layer whose cell adhesiveness is stronger than that of regions having good cell adhesiveness (to which the cells have adhered) on a cell array substrate. For example, the following reference discloses that a cell culture substrate that is desired for stable tissue formation of cells has a soft surface and proper (not too high) adhesiveness (by which cells adhere to the substrate): Mechanochemical Switching Between Growth and Differentiation During Fibroblast Growth Factor-Stimulated Angiogenesis In Vitro: Role of Extracellular Matrix. Donald E. Ingber et al., J. of Cell Biol. (1989) p. 317.

A collagen sheet or the like can be used as a cell culture substrate to which cells can adhere and with which they can be cultured. Moreover, when a cell culture layer (described later) is provided, any material that does not inhibit the culture of cells on the cell culture layer may be used. Examples of such material that can be used herein include, glass, polystyrene, polyethylene terephthalate, polycarbonate, and polyimide. Materials described above for a substrate that is used for a cell array substrate can also be used.

A cell culture layer preferably comprises on its surface a chemical substance or a cell adhesion factor, with which cell adhesion and cell growth can be successfully carried out. Specific examples of such chemical substance or a cell adhesion factor include extracellular matrices such as various types of collagen, fibronectin, laminin, vitronectin, cadherin, gelatin, peptide, and integrin. One type or 2 or more types thereof may be used together. Because of high cell adhesiveness, various types of collagen are more preferably used. Among various types of collagen, type I collagen or type IV collagen is particularly preferably used. A cell culture layer can also be formed by culturing extracellular-matrix-producing cells such as osteoblasts so as to cause the cells to produce extracellular matrices.

The shape of a cell culture substrate is not particularly limited, as long as the substrate is provided with a surface to which cells can be transferred. For example, a culture plate such as a petri dish or a multi-dish plate can be used. Furthermore, a culture plate made of glass or the above plastic can also be used.

Cells can be transferred from a cell adhesion substrate to a cell culture substrate by causing the surface of the cell adhesion substrate to which cells have adhered to come into contact with the surface (e.g., cell culture layer) of the cell culture substrate. Through culture of the cells while maintaining such contact between the cell adhesion substrate and the cell culture substrate, the cells can be transferred. Such culture is carried out generally at a CO₂ concentration of 5% and 37°C for 3 to 96 hours.

Subsequently, the cells are cultured in a culture solution. The cells may be cultured while maintaining such contact between a cell adhesion substrate and a cell culture substrate. Alternatively, the cells may be cultured on the cell culture substrate alone after the removal of the cell adhesion substrate. Preferably, the cells are cultured for a certain time period while maintaining contact between the cell adhesion substrate and the cell culture substrate, the cell adhesion substrate is removed, and then the cells are further cultured. Culture conditions are not particularly limited and can be selected depending on the cell type to be cultured. A culture solution similar to that described above can be used herein.

With the cell adhesion substrate of the present invention, cells adhering in a pattern can be easily transferred to a cell culture substrate while maintaining such patterned state. Furthermore, the cell adhesion substrate is washed after transfer and then can be used as a cell array substrate on which cells can be inoculated again. Thus, such cell pattern can be formed again, transferred, and then cultured. Therefore, unlike conventional technology, there is no need to prepare a culture substrate (having a pattern formed thereon for culturing cells in a pattern) for every culture. Cells can be arrayed in a pattern simply by transferring the cells to a general culture substrate having no such pattern. Accordingly, such cell pattern can be formed efficiently at low cost. Moreover, there is no need to form any special pattern for a cell culture substrate. Thus a substrate that is generally used for culturing cells can be used, so that options for material selection can be expanded. Furthermore, cells to be cultured can be free from the effect of toxic substances such as a developing solution.

An example of the cell culture substrate of the present invention is a biomaterial. The biomaterial means a material derived from a living body and examples of such biomaterial include tissues and organs derived from living bodies. Specific examples of such biomaterial include organs such as lungs, heart, liver, kidney, brain, gaster, small intestine, and large intestine and tissues such as bone, cartilage, skin, muscle, eye, tongue, and peritoneum. Furthermore, a cell sheet and a cell aggregate such as spheroid can also be used as a cell culture substrate. Examples of cells constituting a cell aggregate include extracellular-matrix-producing cells such as stromal cells, epithelial cells, and parenchymal cells. Specifically, aggregates of osteoblasts, fibroblasts, hepatic parenchymal cells, feeder cells, or the like can be preferably used.

Through direct transfer of cells on a cell array substrate to such biomaterial, the cells can be directly cultured in a pattern on such tissue or organ. Examples of a combination of a biomaterial to which cells are transferred and the cells to be transferred to and cultured on the biomaterial include the liver and vascular endothelial cells, the corium and vascular endothelial cells, an osteoblast layer and vascular endothelial cells, a fibroblast layer and hepatic parenchymal cells, an endothelial cell layer and hepatic parenchymal cells, and a feeder cell layer and epithelial cells of the cornea.

In such embodiment, cells can be directly transferred to and cultured on a biomaterial such as an organ. Thus, there is no need to remove and harvest cells from a carrier for cell culture via enzyme treatment or the like as carried out in conventional technology, so that damage to cells can be prevented. As described above, biological tissues and cell tissues formed on organs are also encompassed within the scope of the present invention.

In organ transplantation, capillary vessels are formed on the surface of the organ after transplantation. Technology to effectively carry out transplantation is known, where such capillary vessels are previously formed on the surface of an organ to which the vessels are transplanted and then transplantation is carried out. Specifically, such method according to the conventional technology comprises forming capillary vessels before transplantation and then causing the capillary vessels to adhere to the surface of an organ. However, such method requires much time to form capillary vessels, so that immediate transplantation is impossible. Furthermore, tissue is damaged when vessels previously formed using a culture substrate or the like are removed from the substrate and then transferred to the surface of an organ. According to the method of the present invention, cells arranged in a pattern on a cell adhesion substrate are transferred to the surface of an organ and then transplantation can be carried out without waiting for complete formation of capillary vessels. Thus, the method enables immediate transplantation. Vascular endothelial cells transferred onto the surface of an organ in linear or reticular pattern can easily form tissues, so as to promote *in vivo* formation of capillary vessels. Moreover, according to the present invention, no treatment is required for removing cells from a cell array substrate when the cells are transferred. Thus, no problem such as damage to tissues will arise.

The present invention also relates to a method for regenerating a tissue of a subject, which comprises causing cells derived from a subject to adhere to the cell array substrate of the present invention so as to form a pattern, transferring the cells to a biological tissue of the subject (specifically, transferring the cells in a patterned state onto the surface of an organ, skin, bone, or the like as described above), and then growing the cells.

Examples of such subject are not particularly limited and include mammals. A preferable example is a human. For example, according to the method of the present invention, fibroblasts of the corium or epithelial cells are directly transferred to an injured skin area of a living body and then the cells are grown, so that the skin tissue of the subject can be regenerated. Furthermore, vascular endothelial cells are transferred in a pattern to an injured skin area of a subject and then the cells are grown so as to cause the formation of capillary vessels, so that skin regeneration can also be promoted. Moreover, it also becomes possible to generate a neural circuit or a neural computer by arraying nerve cells in a pattern and culturing the nerve cells.

When transferred cells are cultured, if necessary, a cell stimulating factor is added, so that cellular activity can be enhanced or cells' original functions are caused to be exerted so as to be able to promote tissue formation. As such cell stimulating factor, any substances having activity to promote tissue formation by cells can be used. Examples of such cell stimulating factor include a vascular endothelial cell growth factor (DEGF), a fibroblast growth factor (FGF), a nerve growth factor (NGF), an epidermal growth factor (EGF), and an insulin-like growth factor (IGF).

### Brief Description of the Drawings

Fig. 1 shows an example of a step in the method for producing the cell array substrate of the present invention.
Fig. 2 shows another example of a step in the method for producing the cell array substrate of the present invention.
Fig. 3 shows another example of a step in the method for producing the cell array substrate of the present invention.
Fig. 4 is a schematic sectional view showing an example of the photocatalyst-comprising-layer-side base plate in the present invention.
Fig. 5 is a schematic sectional view showing another example of the photocatalyst-comprising-layer-side base plate in the present invention.
Fig. 6 is a schematic sectional view showing another example of the photocatalyst-comprising-layer-side base plate in the present invention.
Fig. 7 is a schematic view showing an example of the method of the present invention.
Fig. 8 is a schematic view showing an example of the method of the present invention.
Fig. 9 is a photograph showing cells arrayed on a cell array substrate.
Fig. 10 is a photograph showing cell tissues formed according to the present invention.
Fig. 11 is a photograph showing cell tissues formed according to the present invention.
Fig. 12 shows procedures of the method for preparing a cell adhesion substrate according to the present invention.
Fig. 13 shows the state of the cell adhesion substrate at each step in the procedures shown in Fig. 12.
Fig. 14 shows procedures of the method for transferring and culturing cells according to the present invention.
Fig. 15 shows the state of the cell adhesion substrate and that of the cell culture substrate at each step of the procedures shown in Fig. 14.

### Explanation of symbols

- 1: Substrate
- 2: Photocatalyst-comprising cell adhesiveness variation layer
- 3: Substrate for pattern formation
- 4: Photomask
- 5: Energy
- 6: Cell adhesiveness variation pattern
- 15: Cell array substrate
- 16: Cell culture substrate
- 17: Region having good cell adhesiveness
- 18: Region having inhibited cell adhesiveness
- 19: Cell
- 20: Water repellent material
- 21: Cell adhesive material

This description includes part or all of the contents as disclosed in the description and/or drawings of Japanese Patent Application No. 2003-358397, which is a priority document of the present application.

### Best Mode of Carrying Out the Invention

Hereinafter, the present invention will be described in detail by referring to examples, but the present invention is not limited by these examples.

### Example 1

1.5 g of fluoroalkyl silane TSL8233 (GE Toshiba Silicones), 5.0 g of tetramethoxysilane TSL8114 (GE Toshiba Silicones), and 2.4 g of 5.0 × 10⁻³N HCl were mixed for 12 hours and then diluted 10-fold with isopropyl alcohol.

Next, 2.0 g of the solution was applied to a 10 cm × 10 cm soda glass substrate using a spin coater at 1000 rpm for 5 seconds. The substrate was dried at 150°C for 10 minutes.

Next, 3.0 g a titanium oxide sol solution (ISHIHARA SANGYO KAISHA, LTD. STK-03) diluted 3-fold with isopropyl alcohol was used as a composition for a photocatalyst-comprising layer.

The above composition for a photocatalyst-comprising layer was applied to the patterned surface (on which line portions each having a width of 60 µm and space portions each having a width of 300 µm had been arranged alternately) of a line & space negative photomask (quartz) using a spin coater at 700 rpm for 3 seconds, followed by 10 minutes of drying treatment at 150°C. Thus, a photomask comprising a transparent photocatalyst-comprising layer was formed.

The above photocatalyst-comprising layer surface of the photomask and the above cell adhesiveness variation material layer surface of the substrate were arranged with a space of 10 µm between the surfaces. UV exposure was carried out from the photomask side using a mercury lamp (wavelength: 365 nm) with an illuminance of 25.0 mW/cm ² for a predetermined time. The thus obtained cell array substrate had a cell adhesiveness variation pattern wherein linear regions having good cell adhesiveness and each having a width of 60 µm and the spaces comprising regions having inhibited cell adhesiveness and each having a width of 300 µm had been arranged alternately.

Subsequently, the water contact angle at a portion of the cell array substrate subjected to exposure was measured using a contact angle meter (KYOWA INTERFACE SCIENCE CO., LTD.).

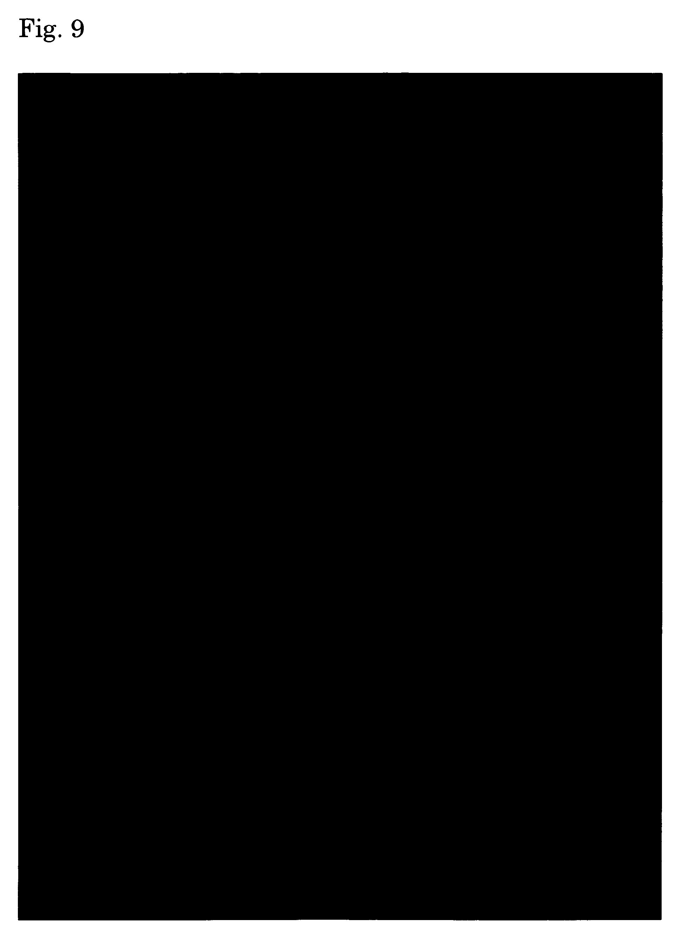

Furthermore, previously-cultured bovine aortic vascular endothelial cells were inoculated on a cell array substrate. Cell adhesiveness to regions having good cell adhesiveness was observed. Fig. 9 shows the result of taking a photograph from above the cell array substrate from above (exposure time: 360 seconds).

The results of measuring the water contact angles and evaluating the cell adhesiveness of a portion subjected to exposure are listed in Table 1 for each exposure time.

**Table 1**

| Exposure time (second) | Water contact angle (°) | Cell adhesiveness |
|---|---|---|
| 0 | 112.5 | × |
| 120 | 62.3 | × |
| 150 | 43.5 | × |
| 180 | 39.8 | Δ |
| 240 | 34.0 | Δ |
| 360 | 23.9 | ○ |
| 480 | 18.7 | ○ |
| 600 | 15.4 | ○ |
| 720 | 13.0 | ×× |
| 900 | *Immeasurable | ×× |

| | | |
|---|---|---|
| × No cells adhered. Δ Cells adhered to form a monolayer with a low density. ○ Cells adhered to form a monolayer with a high density. × Cells adhered in the form of particles without forming a monolayer. Many cells adhered to portions not subjected to exposure. * Immeasurable because of an extremely low water contact angle | | |

Based on the above results, it was revealed that preferable cell adhesiveness can be obtained when the water contact angle is between 10° and 40° in a cell adhesion region.

### Example 2

As cells to be cultured, bovine carotid-derived vascular endothelial cells (Onodera M, Morita I, Mano Y, Murota S: Differential Effects of Nitric Oxide on the Activity of Prostaglandin Endoperoxide h Synthase-1 and-2 in Vascular Endothelial Cells, Prostag Leukotress 62: 161-167, 2000) of 10^{th} to 17^{th} generations obtained by successive culture were used.

Bovine carotid-derived vascular endothelial cells that had reached confluence in a 10 cm dish were removed by 0.05% trypsin-EDTA treatment. The number of cells was counted using a Coulter counter^{™} ZM and then the concentration was adjusted to 10⁶ cells/ml. The cell array substrate (exposure time: 360 seconds) prepared in Example 1 was sterilized with an autoclave. The above endothelial cells were inoculated at 10⁶ cells/5 ml per well on the culture dish (Heraeus Quadriprem^{™}, 76 mm × 26 mm, and 1976 mm ²) on which the cell array substrate had been placed. The cells were incubated for 24 hours using a CO₂ incubator.

0.5 ml to 0.8 ml of a Growth Factor Reduced Matrigel^{™} matrix (BD Biosciences) (hardened at normal temperature) was added dropwise to a new culture dish at 4°C (celsius degree) and then allowed to stand at room temperature for several minutes, thereby preparing a cell culture substrate. The cell array substrate was placed on the cell culture substrate so that the cell-adhered surface of the cell array substrate to which vascular endothelial cells had adhered was caused to come into contact with the above matrix. The resultant was placed within a CO ₂ incubator for 10 minutes. Subsequently, the culture dish was taken out and then 5 ml of a culture solution (MEM medium comprising 5% fetal calf serum) was added thereto, followed by 24 hours of culture. While maintaining such state, the cell array substrate was removed using forceps, then it was cultured for additional 1 to 3 days.

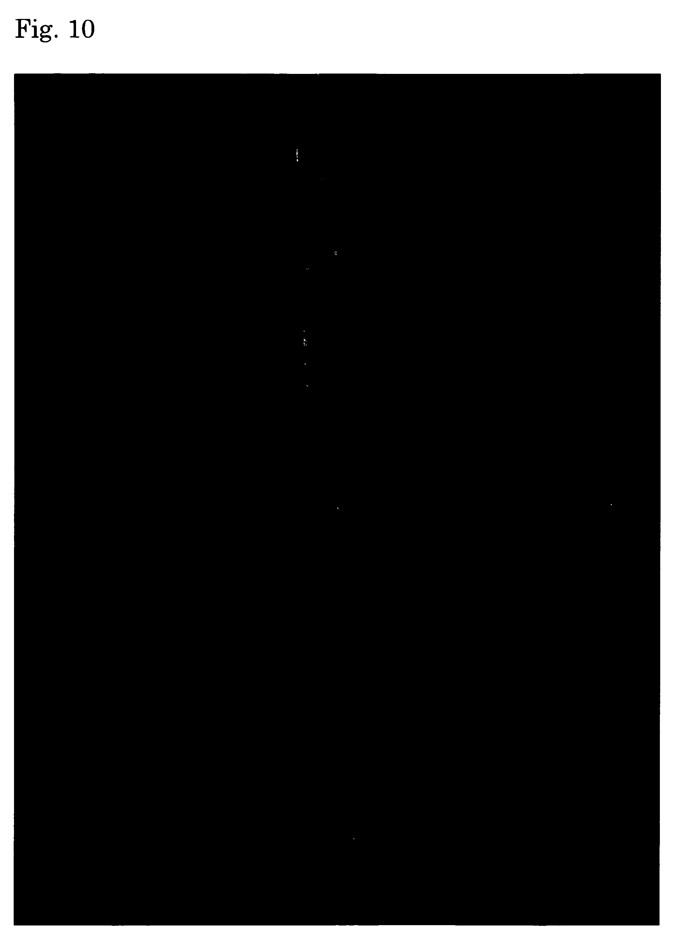

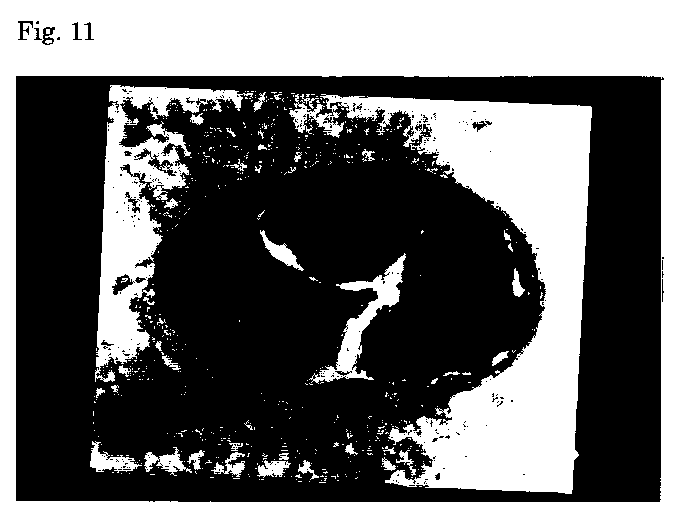

Through observation using a microscope, it could be confirmed that the cells had been arranged in a pattern and that lumen had then been formed. Fig. 10 and Fig. 11 show the results of taking photographs. Fig. 10 is a photograph of cell tissues taken from above the cell culture substrate. Fig. 11 is a photograph of a sectional view of the thus formed vascular tissue tube. The white portion at the center of the tube is "lumen."

### Example 3

10 g of a fluorine coating agent XC98-B2742 (GE Toshiba Silicones) was diluted 10-fold with isopropyl alcohol. 5 g of 1,3-butanediol was further added as a solvent with a high boiling point and then the solution was stirred for 5 minutes.

A polyester film 150-T60 (Lumilar, Toray Industries, Inc.) having a thickness of 150 µm, which had been cut to A4 size, was spin-coated with the solution. Subsequently, the film was heated in a clean oven at 130°C for 10 minutes, washed with water, and then dried at 90°C for 3 minutes.

In the meantime, on a negative photomask (quartz), line portions (opening) each having a width of 60 µm and space portions (shielding portions) each having a width of 300 µm were arranged alternately. Line portions (openings) each having a width of 60 µm orthogonally crossing the line & space pattern were formed at intervals of 2.5 cm. In a manner similar to that of Example 1, the photomask was coated with a photocatalyst layer. Thus, a photocatalyst photomask to be used in this example was prepared.

The photocatalyst photomask was allowed to stand on the coating surface of the above-coated film, so that the photocatalyst surface and the coating surface of the film faced each other. Irradiation of ultraviolet rays (12J · cm⁻²) was carried out from the substrate side of the photomask using an exposure machine, thereby preparing a cell array substrate made of film. Exposure was carried out for 7 minutes. The water contact angle in the regions having good cell adhesiveness of the thus obtained cell array substrate was 36.8°.

### Example 4

Human umbilical vein endothelial cells were collected from the umbilical cord and then cultured (separated using 0.25% trypsin and then cultured). Human umbilical vein endothelial cells of up to the 5^{th} generation obtained by successive culture were used in this example.

The human umbilical vein endothelial cells that had reached confluence in a 10 cm dish were removed by 0.05% trypsin-EDTA treatment. The number of the cells was counted using a Coulter counter^{™} ZM and then the concentration was adjusted to 10 ⁶ cells/ml. The cell array substrate made of film prepared in Example 3 was sterilized with an autoclave. The culture substrate was cut into 1.5 cm × 2.5 cm squares using sterilized forceps, thereby obtaining small sections. At this time, the substrate was cut so that each small section contained a crossing portions (where lines crossed each other) of the pattern of the cell adhesive site. The cell array substrate was arranged on a culture dish (Heraeus Quadriprem^{™}, 76 mm × 26 mm) and then the above endothelial cells were inoculated at 10⁶ cells/5 ml per well, followed by 24 hours of incubation in a CO 2 incubator.

200 µl of a Growth Factor Reduced Matrigel^{™} matrix (BD Biosciences) was added dropwise to another culture dish at 4°C (Celsius degree) and then allowed to stand at room temperature for several minutes, thereby preparing a cell culture substrate. The cell array substrate was allowed to stand on the cell culture substrate so that the cell-adhered surface of the cell array substrate to which human umbilical vein endothelial cells had been caused to adhere on the preceding day and the above matrix faced each other. The resultant was allowed to stand within a clean bench for 2 minutes. 2 ml of a culture solution (RPMI medium comprising 20% fetal calf serum) was added to the resultant and then the cells were cultured for 24 hours. While maintaining such state, only the cell array substrate alone was removed using forceps. The remaining cells were further cultured for 1 to 3 days.

Through observation using a microscope, it could be confirmed that the cells had been arranged in a pattern and that lumen had then been formed. Furthermore, the cell pattern of the crossing portions was also maintained. When a fluorescent dye solution was injected, the intratubal flow of the dye solution could be confirmed.

### Example 5

A cell array substrate was prepared with the same procedures as those in Example 3 except for using a polyester film 25-T60 (Lumilar, Toray Industries, Inc.) having a thickness of 25 µm.

Bovine carotid-derived vascular endothelial cells similar to those used in Example 2 were cultured on the cell array substrate in a manner similar to that in Example 2.

Under sterilization, skin of the dorsal region, peritoneum, and the liver were each excised from a nude mouse (age in days: 5, ♂). Each sample was placed on a 35 mm culture dish. In addition, the subcutaneous tissue was removed from the skin and the serous membrane was removed from the liver. A cell array substrate to which vascular endothelial cells had been caused to adhere was placed on each excised tissue. 3 ml of a culture solution (MEM medium comprising 5% fetal calf serum) was added to each culture dish, and culture was carried out for 48 hours. Each cell array substrate was then removed using forceps. The endothelial cells were retained on excised tissue samples in all experiments. Through observation using a microscope, it could be confirmed that lumen was formed in a pattern on each excised tissue.

### Example 6

Bovine carotid-derived vascular endothelial cells were cultured using a cell array substrate that was the same as that in Example 1 in a manner similar to that in Example 2.

Mouse osteoblast-like cells (MC3T3E1) were inoculated on a new culture dish. When the cells reached confluence and such state was confirmed, the cells were further incubated for 2 or more days. Thus, the production of extracellular matrices was promoted. The cell array substrate was placed on the osteoblasts so that the cell-adhered surface of the cell array substrate to which vascular endothelial cells had been adhered could be adhered to the above osteoblasts. The resultant was allowed to stand within a clean bench for 5 minutes. 5 ml of a culture solution (MEM medium comprising 5% fetal calf serum) was added to the resultant and then the cells were cultured for 48 hours. While maintaining such state, the cell array substrate was removed using forceps, followed by 1 to 3 days of culture.

Through observation using a microscope, it could be confirmed that endothelial cells had been arranged in a pattern on the osteoblast-like cell layer and in the form of lumen.

### Industrial Applicability

According to the present invention, cells can be arrayed and cultured in a fine pattern by a convenient method without experiencing damage.

## Claims

1. An in vitro or ex vivo method for culturing cells, which comprises the steps of: causing cells to adhere to the surface of a cell array substrate having a cell adhesiveness variation pattern that comprises regions having good cell adhesiveness and regions having inhibited cell adhesiveness patterned on a substrate; transferring the adhered cells to a cell culture substrate in such patterned state; and culturing the transferred cells, wherein the regions having good cell adhesiveness in the cell adhesiveness variation pattern have water contact angles between 10° and 40°.

2. The method according to claim 1, wherein the cell adhesiveness variation pattern is formed of a cell adhesiveness variation layer that comprises a cell adhesiveness variation material whose cell adhesiveness is varied by the action of a photocatalyst along with energy irradiation.

3. The method according to claim 2, wherein the cell adhesiveness variation layer is a photocatalyst-comprising cell adhesiveness variation layer that comprises a photocatalyst and the cell adhesiveness variation material.

4. The method according to claim 2, wherein the cell adhesiveness variation layer comprises a photocatalyst-comprising photocatalyst treatment layer and a cell adhesiveness variation material layer that comprises the cell adhesiveness variation material formed on the photocatalyst treatment layer.

5. The method according to claim 2, wherein the cell adhesiveness variation pattern is formed by arranging the cell adhesiveness variation layer that comprises the cell adhesiveness variation material and the photocatalyst-comprising layer so that the layers face each other, and then carrying out energy irradiation.

6. The method according to any one of claims 1 to 5, wherein the cell culture substrate is made of a biomaterial.

7. The method according to any one of claims 1 to 6, wherein the cell adhesiveness variation pattern is a pattern wherein linear regions having good cell adhesiveness arranged on regions having inhibited cell adhesiveness.

8. The method according to any one of claims 1 to 7, wherein the cell adhesiveness variation pattern is a pattern wherein linear regions having good cell adhesiveness and spaces comprised of the regions having inhibited cell adhesiveness are arranged alternately, the line widths of the regions having good cell adhesiveness are each between 20 µm and 200 µm, the space widths between such lines are each between 300 µm and 1000 µm, and the cells used are vascular endothelial cells.

9. A cell adhesion substrate comprising adhered cells for use in a method for regenerating a tissue of a subject, which method comprises transferring said adhered cells, which are cells derived from a subject and caused to adhere to the cell adhesion substrate, onto a biological tissue of the subject in a patterned state and then growing the cells, wherein the cell adhesion substrate comprises a cell array substrate having a cell adhesiveness variation pattern that comprises regions having good cell adhesiveness and regions having inhibited cell adhesiveness patterned on a substrate wherein cells adhere to the regions having good cell adhesiveness in the cell adhesiveness variation pattern in the cell array substrate, and wherein the regions having good cell adhesiveness in the cell adhesiveness variation pattern have water contact angles between 10° and 40°.

10. A cell adhesion substrate comprising adhered vascular endothelial cells for use in a method of treatment, said method comprising transferring said vascular endothelial cells onto the surface of an organ in a patterned state and then transplanting the organ into a subject, wherein the cell adhesion substrate comprises a cell array substrate having a cell adhesiveness variation pattern that comprises regions having good cell adhesiveness and regions having inhibited cell adhesiveness patterned on the substrate wherein cells adhere to the regions having good cell adhesiveness in the cell adhesiveness variation pattern in the cell array substrate, and wherein the regions having good cell adhesiveness in the cell adhesiveness variation pattern have water contact angles between 10° and 40°.

11. The cell adhesion substrate for use according to claim 9 or claim 10, wherein the cell adhesiveness variation pattern is formed of a cell adhesiveness variation layer that comprises a cell adhesiveness variation material whose cell adhesiveness is varied by the action of a photocatalyst along with energy irradiation.

12. The cell adhesion substrate for use according to claim 9 or claim 10, wherein:
the cell adhesiveness variation layer is a photocatalyst-comprising cell adhesiveness variation layer that comprises a photocatalyst and the cell adhesiveness variation material;
wherein the cell adhesiveness variation layer comprises a photocatalyst-comprising photocatalyst treatment layer and a cell adhesiveness variation material layer formed on the photocatalyst treatment layer that comprises the cell adhesiveness variation material; or
wherein the cell adhesiveness variation pattern is formed by arranging the cell adhesiveness variation layer that comprises the cell adhesiveness variation material and the photocatalyst-comprising layer so that the layers face each other, and then carrying out energy irradiation.

## Patentansprüche

1. *In-vitro-* oder *Ex*-*vivo*-Verfahren zur Kultivierung von Zellen, das die folgenden Schritte umfasst: das Verursachen, dass Zellen an der Oberfläche eines Zellarraysubstrats mit einer Zellhaftungsvariationsstruktur haften, die Regionen mit guter Zellhaftung und Regionen mit gehemmter Zellhaftung strukturiert auf einem Substrat umfasst; das Transferieren der haftenden Zellen auf ein Zellkultursubstrat in diesem strukturierten Zustand; und das Kultivieren der transferierten Zellen, worin die Regionen mit guter Zellhaftung in der Zellhaftungsvariationsstruktur Wasserkontaktwinkel zwischen 10° und 40° aufweisen.

2. Verfahren nach Anspruch 1, worin die Zellhaftungsvariationsstruktur aus einer Zellhaftungsvariationsschicht ausgebildet ist, die ein Zellhaftungsvariationsstrukturmaterial umfasst, dessen Zellhaftung durch Einwirkung eines Photokatalysators zusammen mit Energiebestrahlung variiert wird.

3. Verfahren nach Anspruch 2, worin die Zellhaftungsvariationsschicht eine Photokatalysator umfassende Zellhaftungsvariationsschicht ist, die einen Photokatalysator und das Zellhaftungsvariationsmaterial umfasst.

4. Verfahren nach Anspruch 2, worin die Zellhaftungsvariationsschicht eine Photokatalysator umfassende Photokatalysatorbehandlungsschicht und eine Zellhaftungsvariationsmaterialschicht umfasst, die das auf der Photokatalysatorbehandlungsschicht ausgebildete Zellhaftungsvariationsmaterial umfasst.

5. Verfahren nach Anspruch 2, worin die Zellhaftungsvariationsstruktur durch Anordnen der Zellhaftungsvariationsschicht, die das Zellhaftungsvariationsmaterial umfasst, und der Photokatalysator umfassenden Schicht, sodass die Schichten einander zugewandt sind, und anschließendes Anwenden von Energiebestrahlung ausgebildet wird.

6. Verfahren nach einem der Ansprüche 1 bis 5, worin das Zellkultursubstrat aus einem Biomaterial hergestellt ist.

7. Verfahren nach einem der Ansprüche 1 bis 6, worin die Zellhaftungsvariationsstruktur eine Struktur ist, worin lineare Regionen mit guter Zellhaftung auf Regionen mit gehemmter Zellhaftung angeordnet sind.

8. Verfahren nach einem der Ansprüche 1 bis 7, worin die Zellhaftungsvariationsstruktur eine Struktur ist, worin lineare Regionen mit guter Zellhaftung und Räume, die aus den Regionen mit gehemmter Zellhaftung bestehen, abwechselnd angeordnet sind, die Linienbreiten der Regionen mit guter Zellhaftung jeweils zwischen 20 µm und 200 µm betragen, die Raumbreiten zwischen diesen Linien jeweils zwischen 300 µm und 1000 µm betragen und die verwendeten Zellen Gefäßendothelzellen sind.

9. Zellhaftungssubstrat, das haftende Zellen umfasst, zur Verwendung in einem Verfahren zur Neubildung eines Gewebes eines Individuums, wobei das Verfahren das Transferieren der haftenden Zellen, die aus einem Individuum stammende Zellen sind, die dazu gebracht wurden, an dem Zellhaftungssubstrat zu haften, auf ein biologisches Gewebe des Individuums im strukturierten Zustand und das anschließende Züchten der Zellen umfasst, worin das Zellhaftungssubstrat ein Zellarraysubstrat mit einer Zellhaftungsvariationsstruktur umfasst, die Regionen mit guter Zellhaftung und Regionen mit gehemmter Zellhaftung strukturiert auf einem Substrat umfasst, worin Zellen an die Regionen mit guter Zellhaftung in der Zellhaftungsvariationsstruktur in dem Zellarraysubstrat haften und worin die Regionen mit guter Zellhaftung in der Zellhaftungsvariationsstruktur Wasserkontaktwinkel zwischen 10° und 40° aufweisen.

10. Zellhaftungssubstrat, das haftende Gefäßendothelzellen umfasst, zur Verwendung in einem Behandlungsverfahren, wobei das Verfahren das Transferieren der Gefäßendothelzellen auf die Oberfläche eines Organs im strukturierten Zustand und das anschließende Transplantieren des Organs in ein Individuum umfasst, worin das Zellhaftungssubstrat ein Zellarraysubstrat mit einer Zellhaftungsvariationsstruktur umfasst, die Regionen mit guter Zellhaftung und Regionen mit gehemmter Zellhaftung strukturiert auf einem Substrat umfasst, worin Zellen an die Regionen mit guter Zellhaftung in der Zellhaftungsvariationsstruktur in dem Zellarraysubstrat haften und worin die Regionen mit guter Zellhaftung in der Zellhaftungsvariationsstruktur Wasserkontaktwinkel zwischen 10° und 40° aufweisen.

11. Zellhaftungssubstrat zur Verwendung nach Anspruch 9 oder Anspruch 10, worin die Zellhaftungsvariationsstruktur aus einer Zellhaftungsvariationsschicht ausgebildet ist, die ein Zellhaftungsvariationsstrukturmaterial umfasst, dessen Zellhaftung durch Einwirkung eines Photokatalysators zusammen mit Energiebestrahlung variiert wird.

12. Zellhaftungssubstrat zur Verwendung nach Anspruch 9 oder Anspruch 10, worin:
die Zellhaftungsvariationsschicht eine Photokatalysator umfassende Zellhaftungsvariationsschicht ist, die einen Photokatalysator und das Zellhaftungsvariationsmaterial umfasst;
worin die Zellhaftungsvariationsschicht eine Photokatalysator umfassende Photokatalysatorbehandlungsschicht und eine auf der Photokatalysatorbehandlungsschicht ausgebildete Zellhaftungsvariationsmaterialschicht umfasst, die das Zellhaftungsvariationsmaterial umfasst;
worin die Zellhaftungsvariationsstruktur durch Anordnen der Zellhaftungsvariationsschicht, die das Zellhaftungsvariationsmaterial umfasst, und der Photokatalysator umfassenden Schicht, sodass die Schichten einander zugewandt sind, und anschließendes Anwenden von Energiebestrahlung ausgebildet wird.

## Revendications

1. Méthode in vitro ou ex vivo pour la culture de cellules, qui comprend les étapes de : amener les cellules à adhérer à la surface d'un substrat de groupement de cellules ayant un motif de variation d'adhésion cellulaire qui comprend des régions ayant une bonne adhésion cellulaire et des régions ayant une adhésion cellulaire inhibée modelée sur un substrat ; transférer les cellules adhérées sur un substrat de culture de cellules dans un tel état modelé ; et cultiver les cellules transférées, où les régions d'une bonne adhésion cellulaire dans le modèle de variation d'adhésion cellulaire ont des angles de contact avec l'eau entre 10° et 40°.

2. Méthode selon la revendication 1, dans laquelle le modèle de variation d'adhésion des cellules est formé par une couche de variation d'adhésion des cellules qui comprend un matériau de variation d'adhésion des cellules dont l'adhésion des cellules est modifiée par l'action d'un photo-catalyseur conjointement avec une irradiation d'énergie.

3. Méthode selon la revendication 2, dans laquelle la couche de variation d'adhésion des cellules est une couche de variation d'adhésion des cellules comprenant un photo-catalyseur qui comprend un photo-catalyseur et le matériau de variation d'adhésion des cellules.

4. Méthode selon la revendication 2, dans laquelle la couche de variation d'adhésion des cellules comprend une couche de traitement de photo-catalyseur comprenant un photo-catalyseur et une couche de matériau de variation de l'adhésion des cellules qui comprend le matériau de variation d'adhésion des cellules formée sur la couche de traitement de photo-catalyseur.

5. Méthode selon la revendication 2, dans laquelle le modèle de variation de l'adhésion des cellules est formé en agençant la couche de variation d'adhésion des cellules qui comprend le matériau de variation d'adhésion des cellules et la couche comprenant le photo-catalyseur de telle sorte que les couches se font face, et en exécutant ensuite l'irradiation d'énergie.

6. Méthode selon l'une quelconque des revendications 1 à 5, dans laquelle le substrat de culture des cellules est réalisé en un biomatériau.

7. Méthode selon l'une quelconque des revendications 1 à 6, dans laquelle le modèle de variation d'adhésion des cellules est un modèle où des régions linéaires ayant une bonne adhésion cellulaire sont agencées sur des régions ayant une adhésion cellulaire inhibée.

8. Méthode selon l'une quelconque des revendications 1 à 7, dans laquelle le modèle de variation d'adhésion cellulaire est un modèle où des régions linéaires ayant une bonne adhésion cellulaire et des espaces compris des régions ayant une adhésion cellulaire inhibée sont agencées alternativement, les largueurs des lignes des régions ayant une bonne adhésion cellulaire sont chacun entre 20 µm et 200 µm, les largueurs d'espace entre ces lignes sont chacune entre 300 µm et 1000 µm, et les cellules utilisées sont des cellules endothéliales vasculaires.

9. Substrat d'adhésion cellulaire comprenant des cellules adhérentes pour utilisation dans une méthode pour régénérer un tissu d'un sujet, ladite méthode comprend le transfert desdits cellules adhérées, qui sont des cellules dérivées d'un sujet et amenées à adhérer au substrat d'adhésion cellulaire, sur un tissu biologique du sujet en un état modelé, et ensuite la croissance des cellules, où le substrat d'adhésion cellulaire comprend un substrat de groupement de cellules ayant un motif de variation d'adhésion cellulaire qui comprend des régions ayant une bonne adhésion cellulaire et des régions ayant une adhésion cellulaire inhibée modelées sur un substrat, où les cellules adhèrent aux régions ayant une bonne adhésion cellulaire dans le modèle de variation d'adhésion cellulaire dans le substrat de groupement des cellules, et où les régions ayant une bonne adhésion cellulaire dans le modèle de variation d'adhésion cellulaire ont des angles de contact avec l'eau entre 10° et 40°.

10. Substrat d'adhésion cellulaire comprenant des cellules endothéliales vasculaires adhérentes pour utilisation dans une méthode de traitement, ladite méthode comprenant le transfert desdites cellules endothéliales vasculaires sur la surface d'un organe en un état modelé et ensuite la transplantation de l'organe dans un sujet, où le substrat d'adhésion cellulaire comprend un substrat de groupement de cellules ayant un modèle de variation d'adhésion cellulaire qui comprend des régions ayant une bonne adhésion cellulaire et des régions ayant une adhésion cellulaire inhibée sur le substrat, où les cellules adhèrent aux régions ayant une bonne adhésion cellulaire dans le modèle de variation d'adhésion cellulaire dans le substrat de groupement de cellules, et où les régions ayant une bonne adhésion des cellules dans le modèle de variation d'adhésion des cellules ont des angles en contact avec l'eau entre 10° et 40°.

11. Substrat d'adhésion cellulaire pour utilisation selon la revendication 9 ou la revendication 10, où le motif de variation d'adhésion cellulaire est formé par une couche de variation d'adhésion cellulaire qui comprend un matériau de variation d'adhésion cellulaire dont l'adhésion cellulaire varie sous l'action d'un photo-catalyseur conjointement avec une irradiation d'énergie.

12. Substrat d'adhésion cellulaire pour utilisation selon la revendication 9 ou la revendication 10, dans lequel :
la couche de variation d'adhésion cellulaire est une couche de variation d'adhésion cellulaire comprenant un photo-catalyseur qui comprend un photo-catalyseur et le matériau de variation d'adhésion cellulaire ;
où la couche de variation d'adhésion cellulaire comprend une couche de traitement de photo-catalyseur comprenant un photo-catalyseur et une couche de matériau de variation d'adhésion cellulaire formée sur la couche de traitement de photo-catalyseur qui comprend le matériau de variation d'adhésion cellulaire ; ou
où le motif de variation d'adhésion cellulaire est formé en agençant la couche de variation d'adhésion cellulaire qui comprend le matériau de variation d'adhésion cellulaire et la couche comprenant le photo-catalyseur de sorte que les couches se font face, et exécuter ensuite l'irradiation d'énergie.
